# EUROPEAN PATENT APPLICATION

(11) **EP 4 831 177 A1**
(43) Date of publication of application: **28.10.2026**
(21) Application number: 24908076.3
(22) Date of filing: 18.12.2024
(51) Int. Cl.: C07D 487/04, A61K 31/4985, A61P 43/00, A61P 1/16, A61P 11/00, A23L 33/10

(54) **CYCLO-HISPRO DERIVATIVE AND USE THEREOF FOR PREVENTING, AMELIORATING OR TREATING FIBROSIS**

(30) Priority: 18.12.2023 KR 20230185147
(71) Applicant: Novmetapharma Co., Ltd., Seoul 06050 (KR)
(72) Inventor: JUNG, Hoe Yune, Pohang-si, Gyeongsangbuk-do 37668 (KR); EOM, Min Sik, Gwangju 61011 (KR); JEON, Jongsu, Pohang-si, Gyeongsangbuk-do 37664 (KR); BAEK, Seo Yeong, Pohang-si, Gyeongsangbuk-do 37694 (KR); LEE, Do Hyun, Pohang-si, Gyeongsangbuk-do 37667 (KR); LEE, Heon Jong, Incheon 21048 (KR)
(74) Representative: Maiwald GmbH
(86) International application number: PCT/KR2024/020629
(87) International publication number: WO 2025/135793

(57) **Abstract**

The present invention relates to: a cyclo-hispro (CHP) derivative; and a composition for preventing, alleviating or treating fibrosis, the composition comprising same, and more specifically, to a cyclo-hispro derivative in which deuterium is substituted for an imidazole group of cyclo-hispro, and a pharmaceutical composition for preventing or treating fibrosis, a health functional food composition for preventing or alleviating fibrosis, and/or an anti-fibrous composition that include the cyclo-hispro derivative.

## Description

### TECHNICAL FIELD

This application claims priority to and the benefit of Korean Patent Application No. 10-2023-0185147, filed on December 18, 2023, the disclosure of which is incorporated herein by reference in its entirety.

The present invention relates to a cyclo-hispro (CHP) derivative and the use thereof for preventing, ameliorating or treating fibrosis, and more specifically to a cyclo-hispro derivative in which deuterium is substituted on the imidazole group of cyclo-hispro, and a pharmaceutical composition including the same for preventing or treating fibrosis, a health functional food composition for preventing or ameliorating fibrosis, an anti-fibrotic composition and a method for preventing, ameliorating or treating fibrosis using the same.

### BACKGROUND ART

Fibrosis is a disease in which the abnormal production, accumulation and deposition of extracellular matrix by fibroblasts occur, and it is caused by fibrosis of organs or tissues. Fibrosis is a very fatal disease that causes organ damage. For example, idiopathic pulmonary fibrosis (IPF) is a chronic, progressive and fatal disease that occurs as a result of recurrent alveolar epithelial cell damage associated with fibroblast accumulation and myofibroblast differentiation, and causes excessive accumulation of extracellular matrix (ECM) along with the irreversible destruction of lung parenchyma tissue.

Conventional treatment studies have largely targeted the inflammatory process of fibrosis, by using corticosteroids and immunosuppressive drugs. However, these agents have shown little effect in clinical trials, and new drugs to treat fibrosis are needed.

Meanwhile, Korean Patent No. 10-2140910 discloses a composition for preventing, ameliorating or treating fibrosis, including cyclo-hispro, but the anti-fibrotic effect of cyclo-hispro derivatives is unknown.

### DISCLOSURE

### TECHNICAL PROBLEM

An object of the present invention is to provide a novel cyclo-hispro derivative.

Another object of the present invention is to provide a pharmaceutical composition for preventing or treating fibrosis, including a cyclo-hispro derivative.

Still another object of the present invention is to provide a health functional food composition for preventing or ameliorating fibrosis, including a cyclo-hispro derivative.

Still another object of the present invention is to provide an anti-fibrotic composition, including a cyclo-hispro derivative.

Still another object of the present invention is to provide a method for preventing, ameliorating or treating fibrosis using a cyclo-hispro derivative.

Still another object of the present invention is to provide the use of a cyclo-hispro derivative in the manufacture of a medicament or health functional food for preventing, ameliorating or treating fibrosis.

### TECHNICAL SOLUTION

In order to solve the above-described problems, the present invention provides a compound represented by Chemical Formula 1 below, a stereoisomer thereof or a pharmaceutically acceptable salt thereof:

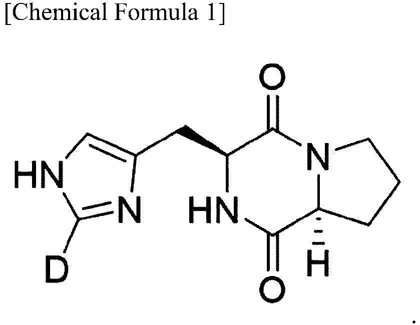

In addition, the present invention provides a pharmaceutical composition for preventing or treating fibrosis, including a compound represented by Chemical Formula 1 above or a pharmaceutically acceptable salt thereof.

Additionally, the present invention provides a health functional food composition for preventing or ameliorating fibrosis, including a compound represented by Chemical Formula 1 above or a sitologically acceptable salt thereof.

In addition, the present invention provides a method for preventing or treating fibrosis, including administering an effective amount of a compound represented by Chemical Formula 1 above or a pharmaceutically acceptable salt thereof to a subject in need thereof.

In addition, the present invention provides a composition for preventing, ameliorating or treating fibrosis, including a compound represented by Chemical Formula 1 above or a pharmaceutically or sitologically acceptable salt thereof to a subject in need thereof.

Additionally, the present invention provides the use of a cyclo-hispro derivative in the manufacture of a medicament or health functional food for preventing, ameliorating or treating fibrosis.

In the present invention, the fibrosis may be any one or more selected from the group consisting of hepatic fibrosis, pulmonary fibrosis, renal fibrosis, dermal fibrosis, cardiac fibrosis, pancreatic fibrosis, neural fibrosis, muscular fibrosis and articular fibrosis.

In the present invention, the hepatic fibrosis may be caused by any one or more selected from the group consisting of chronic liver disease, hepatitis B virus infection, hepatitis C virus infection, hepatitis D virus infection, schistosomiasis, alcoholic liver disease, metabolic dysfunction-associated steatohepatitis, metabolic disease, protein deficiency, coronary artery disease, autoimmune hepatitis, cystic fibrosis, alpha-1 antitrypsin deficiency, primary biliary cirrhosis, drug reaction and toxins.

In the present invention, the pulmonary fibrosis may be caused by any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis, diffuse pulmonary fibrosis, pulmonary edema, cystic fibrosis and metabolic disease.

Furthermore, the present invention provides an anti-fibrotic composition, including a compound represented by Chemical Formula 1 above or a pharmaceutically acceptable salt thereof.

### ADVANTAGEOUS EFFECTS

A composition including a cyclo-hispro derivative of the present invention can effectively inhibit fibrosis of various tissues by inhibiting the activation and expression of fibronectin, α-SMA, collagen (Collagen I, Collagen III, Collagen IV) and inflammatory cytokines (IL-1β, IL-6) by TGF-β, which causes fibrosis.

### DESCRIPTION OF DRAWINGS

FIG. 1 confirms changes in the expression of fibronectin, which is a fibrotic protein, according to treatment with NOV-JD-1 in LX2 cells, which are a human liver cell line.
FIG. 2 confirms changes in the expression of fibronectin, which is a fibrotic protein, according to treatment with NOV-JD-1 in AML12 cells, which are a mouse liver cell line.
FIG. 3 confirms changes in the expression of fibronectin, which is a fibrotic protein, according to treatment with CHP and NOV-JD-1 in LX2 cells, which are a human liver cell line.
FIG. 4 confirms changes in the expression of fibronectin, which is a fibrotic protein, according to treatment with CHP and NOV-JD-1 in AML12 cells, which are a mouse liver cell line.
FIG. 5 measures changes in blood ALT activity in liver function measured by NOV-JD-1 administration in CCl₄-induced liver fibrosis/MASH model animals.
FIG. 6 confirms changes in the gene expression of inflammatory cytokines *IL-1β* (A) and *IL-6* (B) in liver tissues by NOV-JD-1 administration in CCl₄-induced hepatic fibrosis/MASH model animals.
FIG. 7 confirms changes in the expression of fibrotic genes *Collagen I* (A), *Collagen III* (B) and *α-SMA* (C) in liver tissues by NOV-JD-1 administration in CCl₄-induced liver fibrosis/MASH model animals.
FIG. 8 is a diagram measuring changes in hydroxyproline levels in liver tissue by administration of CHP and NOV-JD-1 in CCl₄-induced liver fibrosis/MASH model animals.
FIG. 9 is a diagram confirming changes in the expression of *α-SMA,* which is a fibrotic gene, in liver tissue by administration of CHP and NOV-JD-1 in CCl₄-induced liver fibrosis model animals.
FIG. 10 is a diagram confirming changes in the expression of inflammatory cytokine *IL-1β* (A) and *IL-6* (B) genes in liver tissue by administration of CHP and NOV-JD-1 in CCl₄-induced liver fibrosis model animals.
FIG. 11 is a diagram confirming changes in the expression of fibronectin, which is a fibrotic protein, in liver tissue by administration of CHP and NOV-JD-1 in CCl₄-induced liver fibrosis model animals.
FIG. 12 shows changes in the expression of fibronectin, which is a fibrotic protein, according to treatment with NOV-JD-1 in MRC5 cells, which are a human lung fibroblast cell line.
FIG. 13 shows changes in the expression of Collagen I, which is a fibrotic protein, according to treatment with NOV-JD-1 in primary lung fibroblasts of mice.
FIG. 14 shows changes in the expression of fibronectin, which is fibrotic protein, according to treatment with CHP and NOV-JD-1in primary lung fibroblasts of mice.
FIG. 15 measures changes in hydroxyproline amount in lung tissue by NOV-JD-1 administration in BLM-induced IPF model animals.
FIG. 16 confirms changes in the expression of *Fibronectin* (A), *Collagen I* (B), *Collagen III* (C) and *Collagen IV* (D), which are fibrotic genes, in lung tissues by NOV-JD-1 administration in BLM-induced IPF model animals.
FIG. 17 confirms changes in the expression of fibronectin, which is a fibrotic protein, in lung tissues by NOV-JD-1 administration in BLM-induced IPF model animals.
FIG. 18 confirms changes in the expression of *αSMA* (A) and *Vimentin* (B), which are fibrotic genes, in lung tissues by administration with CHP and NOV-JD-1 in BLM-induced IPF model animals.

### BEST MODE

As described above, conventional fibrosis-related therapeutic studies have largely targeted the inflammatory process of fibrosis using corticosteroids and immunosuppressive drugs, but these agents have shown little effect in clinical trials, and novel drugs for treating fibrosis are needed.

As such, the inventors of the present invention synthesized (3S,8aS)-3-((1H-imidazol-4-yl-2-d)methyl)hexahydropyrrolo[1,2-a]pyrazine-1,4-dione (NOV-JD-1), which is a derivative of cyclo-hispro, and confirmed that the compound is effective in preventing, ameliorating or treating fibrosis by inhibiting the activation and expression of fibronectin, α-SMA, collagen (Collagen I, Collagen III, Collagen IV) and inflammatory cytokines (IL-1β, IL-6) induced by TGF-β, thereby inhibiting fibrosis in various tissues.

Accordingly, the present invention provides a pharmaceutical composition for preventing or treating fibrosis, comprising a compound of Chemical Formula 1 below, which is a cyclo-hispro derivative or a pharmaceutically acceptable salt thereof, or consisting essentially of a compound of Chemical Formula 1 below or a pharmaceutically acceptable salt thereof, or consisting of a compound of Chemical Formula 1 below or a pharmaceutically acceptable salt thereof, and/or a health functional food composition for preventing or ameliorating fibrosis, comprising a compound of Chemical Formula 1 below or a sitologically acceptably salt thereof, or consisting essentially of a compound of Chemical Formula 1 below or a sitologically acceptable salt thereof, or consisting of a compound of Chemical Formula 1 below or a sitologically acceptable salt thereof.

The present invention also provides an anti-fibrotic composition, comprising a compound of Chemical Formula 1 below or a pharmaceutically acceptable salt thereof, consisting essentially of a compound of Chemical Formula 1 below or a pharmaceutically acceptable salt thereof, or consisting of a compound of Chemical Formula 1 below or a pharmaceutically acceptable salt thereof.

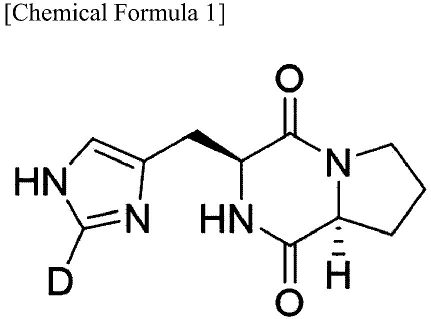

In the present invention, "Cyclo-HisPro (CHP)" refers to a naturally occurring circular dipeptide composed of histidine-proline, which is a metabolite of thyrotropin-releasing hormone (TRH), or a physiologically active dipeptide that is synthesized in the body through TRH metabolism and *de novo*, and is a substance widely distributed throughout the brain, spinal cord and gastrointestinal tract. In addition, Cyclo-HisPro is a compound registered with CAS Number: 53109-32-3, and is also called (3S,8aS)-3-((1H-imidazol-4-yl)methyl)hexahydropyrrolo[1,2-a]pyrazine-1,4-dione.

The inventors of the present invention synthesized (3S,8aS)-3-((1H-imidazol-4-yl-2-d)methyl)hexahydropyrrolo[1,2-a]pyrazine-1,4-dione (NOV-JD-1), which is a cyclo-hispro derivative in which deuterium is substituted on the imidazole group of cyclo-hispro, according to the following reaction scheme, and named it "NOV-JD-1".

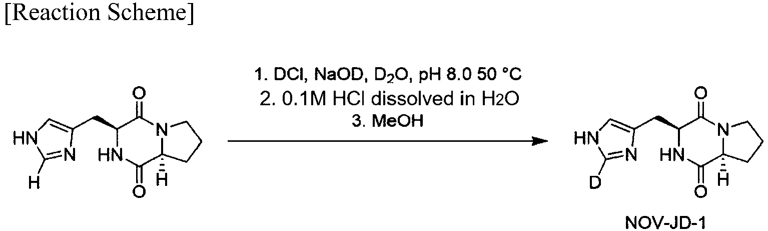

In the present invention, the term "isomer" means a compound of the present invention or a salt thereof which has the same chemical formula or molecular formula but is structurally or sterically different. Such isomers may include racemates, enantiomers, diastereomers, mixtures of enantiomers or mixtures of diastereomers.

In the present invention, the term "fibrosis" is used interchangeably with "fibrotic condition", "fibroproliferative condition", "fibrotic disease", "fibroproliferative disease", "fibrotic disorder" and "fibroproliferative disorder", and refers to a condition, disease or disorder characterized by dysregulated proliferation or activity of fibroblasts, abnormal accumulation of fibronectin, and/or pathological or excessive accumulation of collagenous tissue. Typically, such a condition, disease or disorder is treatable by the administration of a compound having anti-fibrotic activity.

In the present invention, the fibrosis may occur in any one or more selected from the group consisting of, for example, the liver, lungs, kidneys, skin, heart, pancreas, urinary system, reproductive system, sweat glands, nerves, brain, bone marrow, muscles and joints. Specifically, the fibrosis may be any one or more selected from the group consisting of, but is not limited to, hepatic fibrosis, pulmonary fibrosis, renal fibrosis, dermal fibrosis, cardiac fibrosis, pancreatic fibrosis, neural fibrosis, muscular fibrosis and articular fibrosis.

For example, the fibrosis of the present invention may be any one or more selected from the group consisting of pulmonary fibrosis, idiopathic pulmonary fibrosis, radiation-induced lung injury or pulmonary fibrosis, pulmonary edema, cystic fibrosis, liver fibrosis, endomyocardial fibrosis, myocardial infarction, atrial fibrosis, glial scar, renal fibrosis, myelofibrosis, arthrofibrosis, lipofibrosis, skin fibrosis, nerve fibrosis and muscle fibrosis, but is not limited thereto.

More specifically, the cyclo-hispro derivative (NOV-JD-1) according to the present invention exhibits therapeutic effects on various fibrosis diseases described herein.

In the use of the composition of the present invention for preventing, ameliorating or treating fibrosis, a first aspect relates to liver fibrosis, which is fibrosis occurring in the liver.

The term "liver fibrosis" in the present invention refers to a symptom in which fibrous tissue proliferates due to chronic damage to the liver, and may include any one or more selected from the group consisting of chronic liver disease, hepatitis B virus infection, hepatitis C virus infection, hepatitis D virus infection, schistosomiasis, alcoholic liver disease, steatohepatitis associated with metabolic disorders, metabolic diseases, protein deficiencies, coronary artery disease, autoimmune hepatitis, cystic fibrosis, alpha-1 antitrypsin deficiency, primary biliary cirrhosis, drug reactions and toxins, and the liver fibrosis may include steatohepatitis associated with metabolic disorders, but is not limited thereto.

Liver fibrosis is a precursor lesion of cirrhosis, and is initiated by the action of various cytokines and growth factors as a result of severe liver damage that causes chronic liver disease. In general, liver fibrosis is reversible and consists of thin fibrils, and if nodules are not formed and the cause of liver damage is temporary, the extracellular matrix (ECM) increased by apoptosis and matrix metalloproteinases (MMPs) is decomposed, thereby allowing normal recovery. However, if the liver fibrosis process is repeated and persistent, thick fibrils are formed, and it progresses to cirrhosis with nodules. In addition, cirrhosis is induced through the process of liver fibrosis in which hepatocytes are damaged by various inflammatory factors, accumulating abnormal extracellular matrix proteins, including collagen. It is important to control the accumulation of extracellular matrix to control the development of cirrhosis. When hepatocytes are damaged, the inflammatory response activates resting hepatic stellate cells to secrete extracellular matrix and various cytokines and chemokines, among which TGF-β1 acts as a potent growth inhibitor. TGF-β1 is a 25 kD substance that binds to latent TGF-β1 binding protein and is secreted in an inactive latent form, and exists in a bound state with extracellular matrix such as type 1,4 collagen, laminin and decorin, and is activated by various stimuli. TGF-β1 regulates collagen expression by decreasing collagenase production or increasing collagenase inhibitor production, and plays an important role in the fibrosis process by increasing the production of TNF-α, IL-1 and PDGF in macrophages. Currently, TGF-β1 is known to play an important role in liver fibrosis, as it is expressed only in areas where fibrosis has progressed and not in normal liver tissue or inactive areas.

Meanwhile, metabolic dysfunction-associated steatotic liver disease (MASLD) was previously called nonalcoholic fatty liver disease (NAFLD). It can be caused by obesity, diabetes, hyperlipidemia, drugs and the like, regardless of drinking, and refers to a wide range of diseases including simple fatty liver disease (steatosis) without inflammatory response, metabolic dysfunction-associated steatohepatitis (MASH) that shows hepatocellular inflammation, advanced fibrosis and liver cirrhosis as it progresses. The metabolic dysfunction-associated steatohepatitis (MASH) was also previously called non-alcoholic steatohepatitis (NASH).

Metabolic fatty liver disease (MASLD) is a disease that increases in adults due to the consumption of high-fat and high-calorie diets in modern society. It is reported that 20 to 30% of the adult population in developed countries suffer from MASLD, and 2 to 3% develop metabolic steatohepatitis (MASH) patients. In particular, histological findings of steatohepatitis accompanied by fibrosis and inflammation are shown, which greatly increases the risk of developing cirrhosis, liver failure and liver cancer.

Fatty liver is not a pathological condition in itself, and is a reversible symptom that naturally recovers when the causative agent is removed. However, if the state of excessive fat accumulation in the liver tissue continues, steatohepatitis occurs, and as a result, hepatocyte necrosis and regeneration occur repeatedly, and in the process, the fibrous extracellular matrix (ECM) increases, and liver fibrosis progresses. When liver damage reaches a certain stage, the amount of ECM accumulated increases regardless of the type of causative agent, and regenerative nodules are formed as a result of continuous hepatocyte destruction and regeneration, which worsens into irreversible liver cirrhosis.

Therefore, in the present invention, liver fibrosis is a disease that is clearly distinct from fatty liver, which is a reversible symptom, and refers to all diseases in which liver tissue is changed into fibrotic tissue such as regenerative nodules, thereby deteriorating liver function.

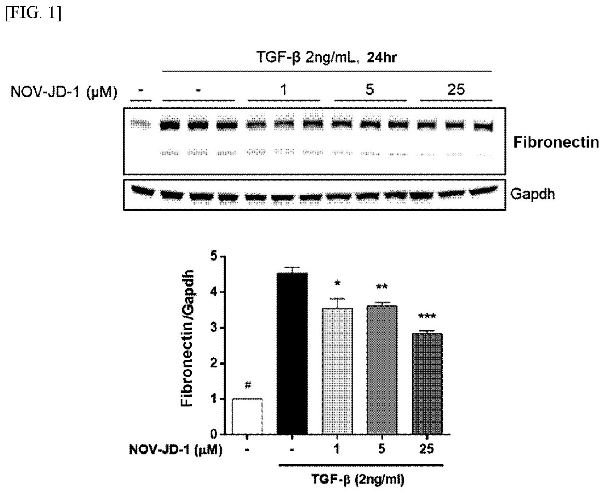

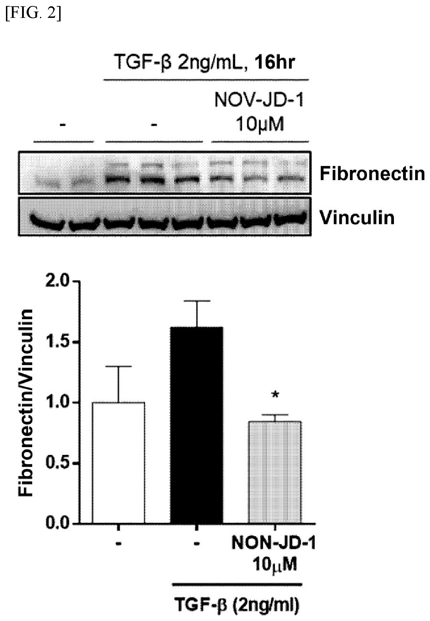

In one embodiment of the present invention, fibrosis was induced in human and mouse liver cell lines using TGF-β, and then, the fibrosis inhibitory effect was evaluated by treating with (3S,8aS)-3-((1H-imidazol-4-yl-2-d)methyl)hexahydropyrrolo[1,2-a]pyrazine-1,4-dione (NOV-JD-1), which is a novel synthesized cyclo-hispro derivative. As a result, as confirmed in FIGS. 1 and 2, the expression of fibronectin, which is a fibrosis protein increased by TGF-β, was significantly reduced by the treatment with NOV-JD-1.

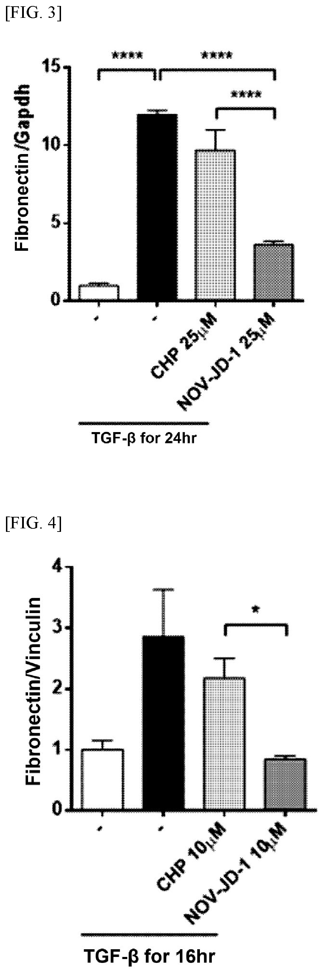

In another embodiment of the present invention, to compare the anti-fibrotic effects of cyclo-hispro and its derivative, NOV-JD-1, in liver cells, fibrosis was induced in a liver cell line using TGF-β, and then, cyclo-hispro and NOV-JD-1 were treated at the same concentration to evaluate the anti-fibrotic effects. As a result, as confirmed in FIGS. 3 and 4, the expression of fibronectin, which is a fibrotic protein increased by TGF-β, was decreased by both cyclo-hispro and NOV-JD-1 treatment, and in particular, the decreasing effect by NOV-JD-1 was significantly superior to that by CHP.

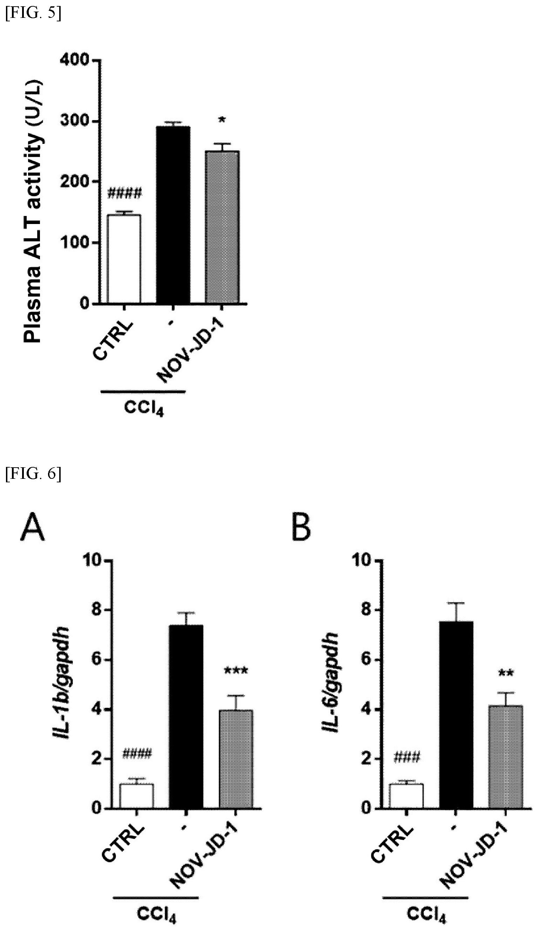

In still another embodiment of the present invention, the effect of NOV-JD-1 administration on improving liver function and inhibiting fibrosis was evaluated in a liver fibrosis/MASH animal model induced using carbon tetrachloride (CCl₄). As a result, as confirmed in FIGS. 5 to 7, in the carbon tetrachloride administration group, ALT (Alanine transaminase) increased due to liver damage caused by carbon tetrachloride, and the expression of inflammatory (*IL-1β, IL-6*) and fibrotic (*Collagen I, Collagen III, α-SMA*) genes in liver tissue significantly increased. However, the administration of NOV-JD-1 significantly decreased ALT, restored liver function, and significantly decreased the expression of inflammatory (*IL-1β, IL-6*) and fibrotic (*Collagen I, Collagen III, α-SMA*) genes.

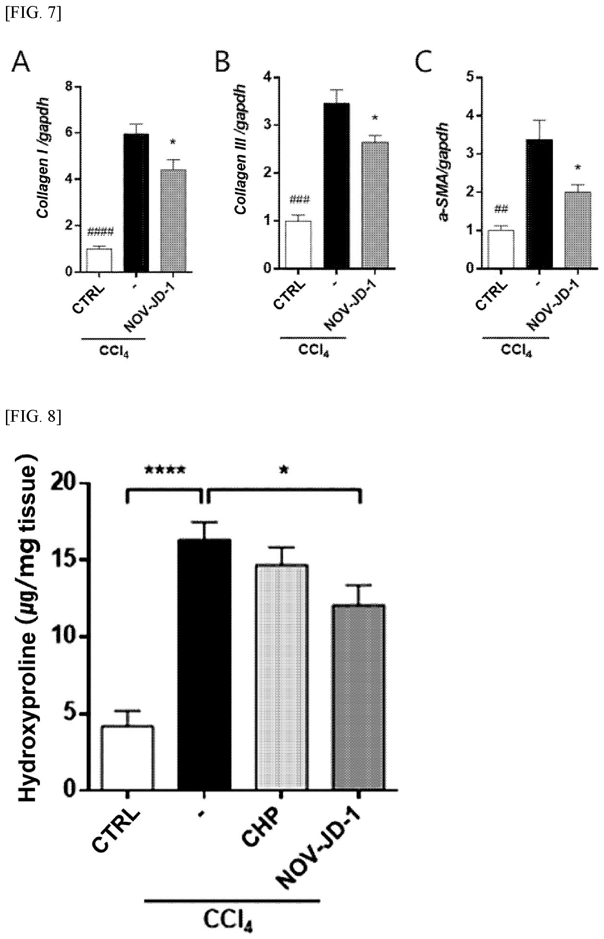

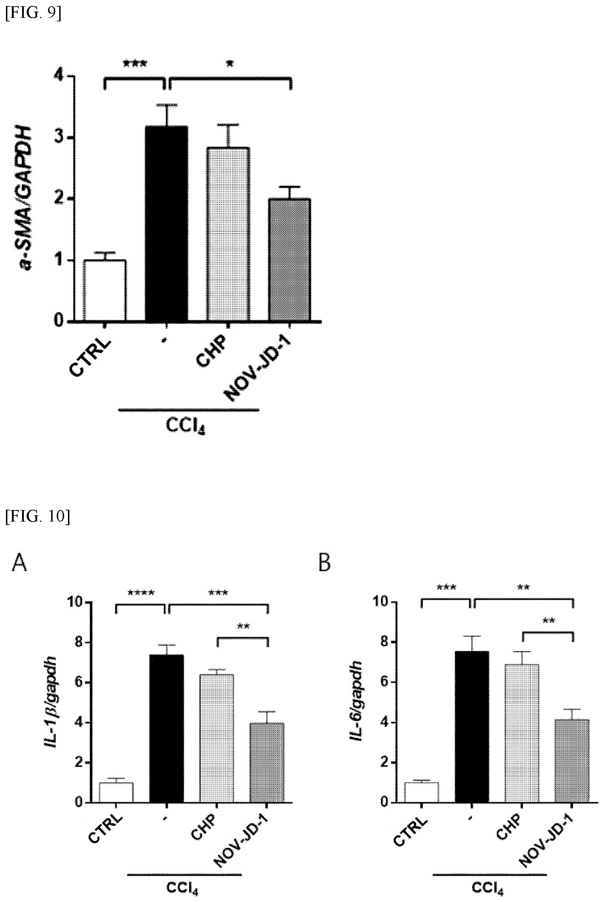

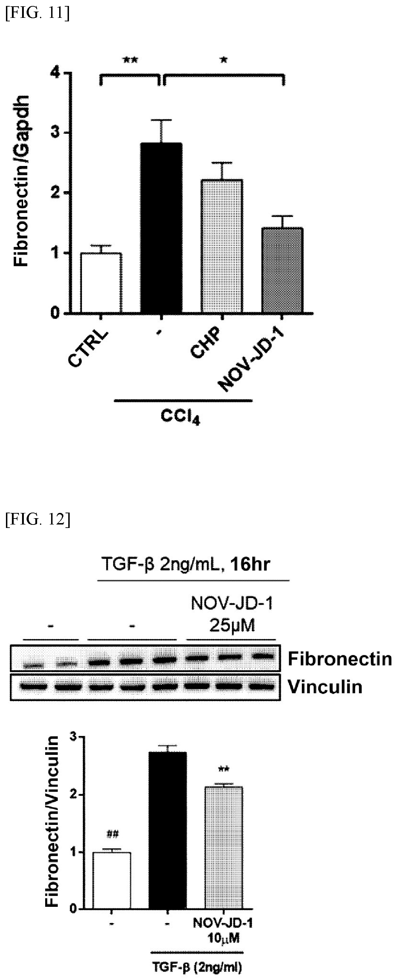

In still another embodiment of the present invention, cyclo-hispro and NOV-JD-1 were administered at the same amount to each other in a liver fibrosis/MASH animal model induced using carbon tetrachloride (CCl₄) to compare their inhibitory effects on fibrosis. As a result, as confirmed in FIG. 8, the hydroxyproline level, which was rapidly increased by CCl₄, was reduced by both cyclo-hispro and NOV-JD-1 treatment, and in particular, the reducing effect by NOV-JD-1 was significantly superior to that by CHP. In addition, as confirmed in FIGS. 9 and 10, the expression of the fibrosis gene *α-SMA* and the inflammatory cytokine genes *IL-1β* and *IL-6*, which were increased by CCl₄, were reduced by both cyclo-hispro and NOV-JD-1 treatment, and in particular, the reducing effect by NOV-JD-1 was significantly superior to that by CHP. Furthermore, as confirmed in FIG. 11, the expression of fibronectin, which is a fibrotic protein increased by CCl₄, was decreased by both cyclo-hispro and NOV-JD-1 treatment, and in particular, the decreasing effect by NOV-JD-1 was significantly superior to that by CHP.

As demonstrated in FIGS. 1 and 2, it can be seen that NOV-JD-1 has an excellent effect in reducing the expression level of fibronectin protein, which is a fibrotic protein, in liver cells induced with fibrosis. In particular, as demonstrated in FIGS. 3 and 4, NOV-JD-1 has a significantly better effect in reducing the expression of fibronectin protein than cyclo-hispro in liver cells induced with fibrosis. In addition, as demonstrated in FIGS. 5 to 7, NOV-JD-1 has an excellent effect in improving liver function and reducing the expression of inflammatory genes and fibrotic genes in an animal model induced with liver fibrosis/MASH. In particular, as demonstrated in FIGS. 8 to 11, NOV-JD-1 reduces the expression of fibrotic genes and inflammatory genes more significantly than cyclo-hispro in an animal model induced with liver fibrosis/MASH, and reduces the expression of fibronectin protein more significantly. Accordingly, NOV-JD-1 exhibits fundamental anti-fibrotic effects and can be applied to treat various liver fibrosis as well as liver failure and liver cancer progressing from cirrhosis regardless of the cause of fibrosis.

In the use of the composition of the present invention for preventing, ameliorating or treating fibrosis, a second aspect relates to pulmonary fibrosis, which is fibrosis occurring in the lungs.

Among fibrosis, pulmonary fibrosis in particular is a disease in which chronic inflammatory cells infiltrate the alveolar walls of lung tissue, thereby inducing tissue fibrosis and causing serious structural changes in lung tissue. Once fibrosis progresses due to any cause, lung tissue hardens and alveolar walls thicken, thereby reducing the amount of oxygen supplied by the blood, making breathing difficult. Currently, there is no treatment method in medicine that can completely restore lung tissue that has already progressed to fibrosis, and thus, unless it is discovered in the early stages of fibrosis or a lung transplant is performed, patients usually die 3 to 5 years after the onset of symptoms.

Specifically, the term "pulmonary fibrosis" in the present invention refers to the development of scarred (fibrous) tissue due to the formation or development of excessive fibrous connective tissue (fibrosis) in the lungs. Specifically, pulmonary fibrosis is a chronic disease that causes swelling and scarring of the alveoli and interstitial tissue of the lungs. This scar tissue replaces healthy tissue, thereby causing inflammation, and chronic inflammation may be identified as a precursor to fibrosis. Such damage to the lung tissue may cause the lungs to become stiff, and the subjects may have difficulty breathing on their own.

In the present invention, pulmonary fibrosis may be caused by any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung, desquamative interstitial pneumonia, lymphoid interstitial pneumonia, interstitial pulmonary fibrosis and diffuse pulmonary fibrosis, pulmonary edema, cystic fibrosis and metabolic disease, but is not limited thereto.

The pulmonary fibrosis may be caused by various causes, such as microscopic damage to the lungs induced by inhalation of fine particles (asbestos, stone dust, metal dust, particles present in cigarette smoke, silica dust, etc.). In addition, pulmonary fibrosis may occur as a secondary effect of other diseases (autoimmune diseases, viral or bacterial infections, etc.), and may be caused by certain drugs such as cytotoxic agents (bleomycin, busulfan, and methotrexate, etc.); antibiotics (nitrofurantoin and sulfasalazine, etc.); antiarrhythmic drugs (amiodarone and tocainide, etc.); antiinflammatory drugs (gold and penicillamine, etc.); and illicit drugs (narcotics, cocaine, and heroin, etc.). In the case of the idiopathic pulmonary fibrosis, it may appear due to unknown causes other than these causes. In addition, long-term intake of a high-fat diet induces pulmonary fibrosis, which seems to be due to an increase in the level of inflammation caused by high-fat intake.

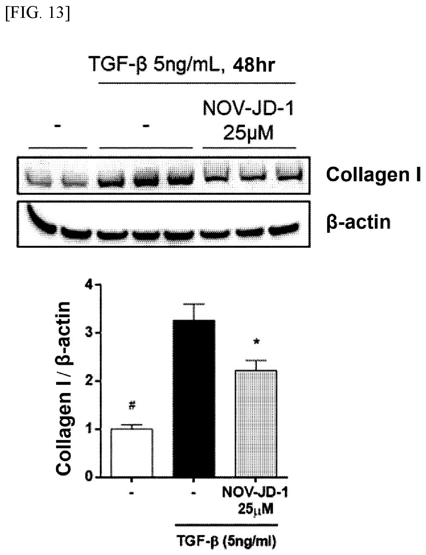

In one embodiment of the present invention, fibrosis was induced using TGF-β in human and mouse lung fibroblast cell lines, and then, the fibrosis inhibitory effect was evaluated by treating with a novel synthesized cyclo-hispro derivative, NOV-JD-1. As a result, as confirmed in FIGS. 12 and 13, the expression of fibrotic proteins fibronectin and Collagen I, which are increased by TGF-β, was significantly reduced by the treatment with NOV-JD-1.

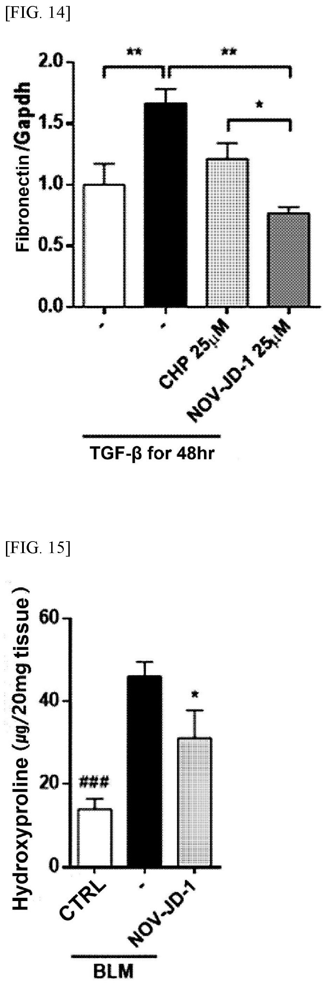

In another embodiment of the present invention, fibrosis was induced in a mouse lung fibroblast cell line using TGF-β, and then, cyclo-hispro and its derivative NOV-JD-1 were treated at the same concentration, respectively, to compare the fibrosis inhibitory effects. As a result, as confirmed in FIG. 14, the expression of fibronectin, which is a fibrosis protein increased by TGF-β, was decreased by both cyclo-hispro and NOV-JD-1 treatment, and in particular, the decreasing effect by NOV-JD-1 was shown to be more remarkable than that by cyclo-hispro.

In still another embodiment of the present invention, the inhibitory effect of NOV-JD-1 administration on fibrosis was evaluated in an idiopathic pulmonary fibrosis (IPF) animal model induced using bleomycin. As a result, as confirmed in FIGS. 15 to 17, in the bleomycin-administered group, the hydroxyproline level rapidly increased, and the expression of fibrotic genes (*Fibronectin*, *Collagen I, Collagen III, Collagen IV* and *α-SMA*) and proteins (Fibronectin) in lung tissue significantly increased, but the administration of NOV-JD-1 significantly decreased the hydroxyproline level, and the expressions of fibrotic genes (*Fibronectin*, *Collagen I, Collagen III, Collagen IV* and *α-SMA*) and proteins (Fibronectin) were significantly reduced.

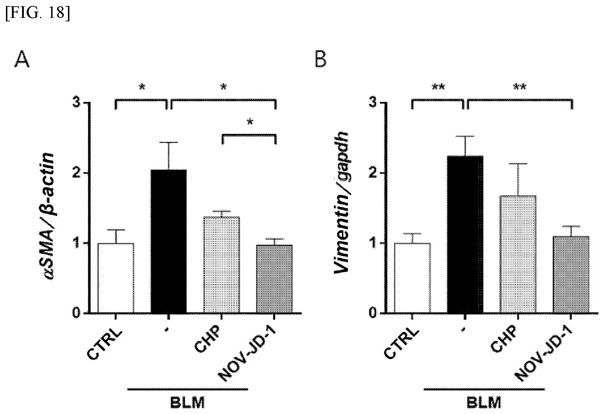

In still another embodiment of the present invention, cyclo-hispro and its derivative NOV-JD-1 were administered in the same amount to an IPF animal model induced using bleomycin, and the fibrosis inhibitory effects were compared. As a result, as confirmed in FIG. 18, the expressions of fibrosis genes *αSMA* and *Vimentin* increased by BLM were both reduced by the administration of cyclo-hispro and NOV-JD-1, and in particular, the reduction effect by NOV-JD-1 was shown to be far superior to that by cyclo-hispro.

As demonstrated in FIGS. 12 and 13, NOV-JD-1 has an excellent effect in reducing the expression levels of fibrotic proteins, Fibronectin and Collagen I protein, in fibrosis-induced lung fibroblasts. In particular, as demonstrated in FIG. 14, it can be seen that NOV-JD-1 significantly reduces the expression of fibrotic protein Fibronectin compared to cyclo-hispro in fibrosis-induced lung fibroblasts. In addition, as demonstrated in FIGS. 15 to 17, NOV-JD-1 has an excellent effect in reducing the hydroxyproline level and the expression of major fibrotic genes (*Fibronectin*, *Collagen I, Collagen III* and *Collagen IV*) and protein (Fibronectin) in an animal model in which pulmonary fibrosis is induced. In particular, as demonstrated in FIG. 18, NOV-JD-1 was found to be more effective in reducing the expression of fibrotic genes (*αSMA* and *Vimentin*) than cyclo-hispro in an animal model in which pulmonary fibrosis was induced. Accordingly, NOV-JD-1 exhibits a fundamental anti-fibrotic effect, and thus can be applied to treat various pulmonary fibrosis regardless of the cause of fibrosis.

In the use of the composition of the present invention for preventing, ameliorating or treating fibrosis, a third aspect relates to renal fibrosis, which is fibrosis occurring in the kidney.

Depending on the progression, kidney disease is classified into acute renal failure or chronic renal failure, and depending on the cause, it is divided into glomerular nephritis caused by deposition of vascular complexes, diabetic nephropathy accompanying diabetes, hypertensive nephropathy accompanying hypertension, toxic nephropathy caused by administration of drugs such as antibiotics or anticancer agents, and bacterial infection. Regardless of the type of kidney disease causing the disease, if renal function impairment progresses chronically and the glomerular filtration rate decreases below 50%, in most cases the glomerular filtration rate continues to decrease, ultimately reaching end-stage renal failure and causing complications such as hematological abnormalities, neurological complications, gastrointestinal complications, immunological complications, infections or osteodystrophy, which may lead to death in severe cases.

Chronic renal failure is a state in which renal function gradually declines unidirectionally (irreversibly), and the body's homeostasis cannot be maintained. All renal diseases are accompanied by renal fibrosis and eventually lead to end-stage renal failure. In particular, chronic renal function decline is deeply related to the progression of renal fibrosis, and thus, suppressing the progression of fibrosis may lead to suppressing the progression of chronic renal failure.

The term "renal fibrosis" in the present invention includes all diseases in which fibrosis occurs in the kidney due to various causes, and the fibrosis may include, but is not limited to, any one or more selected from the group consisting of catheter placement, glomerulosclerosis, glomerulonephritis, nephritis, acute renal failure, chronic renal failure, end-stage renal disease and metabolic disease.

The types of the nephritis include, for example, any interstitial nephritis, for example, poststreptococcal glomerulonephritis, staphylococcal nephritis, pneumococcal nephritis, viral nephritis associated with smallpox, hepatitis B, hepatitis C, HIV and the like, viral nephritis caused by parasitic infections such as malaria, fungal nephritis, infectious interstitial nephritis associated with mycoplasma nephritis and the like, interstitial nephritis associated with collagen diseases such as systemic erythematosus (lupus nephritis), systemic scleroderma (collagen disease kidney) and Sjgren's syndrome, nephritis associated with vascular immune diseases such as purpura nephritis, polyarteritis and rapidly progressive glomerulonephritis, nephritis associated with radiation exposure, drug-induced interstitial nephritis caused by gold preparations, NSAIDs, anticancer agents such as penicillamine and bleomycin, antibiotics and paraquat, allergic nephritis caused by insect bites, pollen, plants of the poison ivy family and the like, amyloidosis nephritis, nephritis associated with diabetic nephropathy, chronic glomerulonephritis, malignant nephrosclerosis and multifocal cystic renal failure, tubulointerstitial nephritis, nephritis associated with toxaemia of pregnancy or cancer, idiopathic interstitial nephritis such as membranous proliferative glomerulonephritis, IgA renal failure, mixed cryoglobulinemia nephritis, Goodpasture's syndrome nephritis, Wegener's granulomatosis nephritis and acute interstitial nephritis, but are not limited thereto.

In the use of the composition of the present invention for preventing, ameliorating or treating fibrosis, a fourth aspect relates to skin fibrosis.

The term "dermal fibrosis" in the present invention refers to excessive scarring of the skin, resulting from a pathological wound healing response. There are a wide range of fibrous skin diseases: scleroderma, nephrosclerotic dermatitis, mixed connective tissue disease, sclerosing myxedema, sclerosing edema and eosinophilic fasciitis. Exposure to chemical or physical agents (mechanical trauma, burn wounds) is also a potential cause of fibrous skin diseases. Dermal fibrosis may be driven by immune, autoimmune and inflammatory mechanisms. The balance of collagen production and degradation by fibroblasts plays an important role in the pathophysiology of dermal fibrosis. Certain cytokines, such as transforming growth factor-β (TGF-β) and interleukin-4 (IL-4), promote wound healing and fibrosis. Fibroblasts in normal skin are quiescent. They regulate the amount of connective tissue proteins and have low proliferative activity. After skin damage, these cells become activated, that is, they express α-smooth muscle actin (α-SMA) and synthesize large amounts of connective tissue proteins. The activated cells are often called myofibroblasts.

The term "dermal fibrosis" in the present invention also means "scleroderma."

Scleroderma is a chronic autoimmune connective tissue disease of unknown etiology characterized by sclerotic changes and vascular abnormalities in which the skin becomes hard and thick in part or all over the body due to excessive accumulation of collagen in the dermis. Collagen forms connective tissue and supports and connects the body's tissues. Scleroderma has several forms, some of which only appear in certain parts of the body, while others appear throughout the body, including internal organs. That is, it is divided into localized scleroderma, in which only a part of the skin becomes hard, and systemic scleroderma, in which fibrotic symptoms due to increased collagen are shown not only in the skin but also in internal organs such as the lungs, digestive system, kidneys and heart. Systemic scleroderma is again classified into limited and extensive types based on the degree of skin and internal organ involvement, prognosis and immunological test results.

In the use of the present invention for preventing, ameliorating or treating skin fibrosis, the skin fibrosis may include, but is not limited to, any one or more selected from the group consisting of scars, hypertrophic scars, keloid scars, localized scleroderma and systemic sclerosis.

The cause of scleroderma is not yet clearly known, but tissue fibrosis is thought to play an important role.

The early symptoms of scleroderma vary widely, but skin symptoms become more prominent in the later stages. Common symptoms of scleroderma include joint pain, morning stiffness, fatigue and weight loss. In addition, exposure to cold temporarily reduces blood flow to the fingers, toes, nose and ears. This is one of the early and most common symptoms of scleroderma. Patients with scleroderma have hardened skin. This hardening is widespread and typically occurs on both sides of the body. Eventually, tissue damage occurs, and the skin becomes pigmented.

"Localized scleroderma" is 2.6 times more common in women than in men. 75% of patients with localized scleroderma develop between the ages of 20 and 50, while pre-existing scleroderma tends to develop at an earlier age. Localized scleroderma is classified as localized scleroderma, systemic localized scleroderma, pre-existing localized scleroderma or subcutaneous localized scleroderma.

Localized scleroderma often starts as erythema or reddish purple patches, and the border with the surrounding normal skin becomes distinct, and the skin in a certain area loses elasticity and hardens. The area becomes brown or, in many cases, discolored and white. Most of the time, it occurs in one place, but sometimes it occurs in multiple places, and the size varies greatly, from the size of a coin to the size of an adult's palm. It causes cosmetic problems, but it does not progress to systemic sclerosis. In some patients, the hard skin softens and recovers on its own without treatment.

Systemic sclerosis is a severe form of localized scleroderma, in which large areas of skin become hardened and hyperpigmentation is observed. The skin on the trunk, buttocks and legs becomes hardened extensively. However, it is distinguished from systemic sclerosis because it does not have Raynaud's phenomenon or internal organ involvement.

Localized scleroderma is a condition in which the skin hardens in a linear manner, and it often occurs on the legs, arms, forehead and chest in that order. In particular, when it occurs in a vertical depression on the forehead, it is called en coup de sabre and causes a cosmetic problem. Unlike localized scleroderma, linear scleroderma affects not only the skin but also the underlying muscles and periosteum, and becomes fixed to the underlying tissue. It occurs frequently in children and may sometimes cause severe skeletal abnormalities in the limbs and face.

Subcutaneous localized scleroderma is characterized by sclerosis of the fat layer, fascia, muscles and sometimes bones, and may limit joint movement. Since the lesions are deep, the characteristic skin pigmentation changes of scleroderma are not observed.

Systemic sclerosis occurs four times more frequently in women than in men, and may occur at any age, but is most common between the ages of 30 and 50. The extensive type is known to occur at a younger age than the limited type. Raynaud's phenomenon is the first symptom to appear in systemic sclerosis, and sclerosis symptoms in the skin and internal organs begin soon after. Systemic sclerosis is classified into skin symptoms and internal organ symptoms.

In systemic sclerosis, the first skin symptoms start in the fingers and hands. At first, only Raynaud's phenomenon is visible, but the fingers and hands become swollen, stiff and red. The skin gradually hardens and spreads to the arms and face, and in the extensive form, the skin of the entire body, including the trunk, becomes hard continuously. When sclerosis occurs on the face, wrinkles disappear overall, and it becomes difficult to make facial expressions. The nose appears pointed, it is difficult to open the mouth, the lips become thin, and radial wrinkles appear around the mouth, resembling the mouth of an old person. The movement of the joints is limited, making it impossible to fully grasp the hand, and the fingers become bent and pointed, showing sclerotherapy of the fingers and toes. Painful ulcers that do not heal well develop on the fingertips and joints.

Widespread hyperpigmentation and localized hypopigmentation may occur, with hair loss and decreased sweating in the affected areas. Telangiectasias may appear as round spots on the face and upper trunk or around the nail folds. In patients with Raynaud's disease, abnormalities in the capillaries of the nail folds may lead to subsequent systemic sclerosis, and thus, capillary changes are important in determining the prognosis. Skin calcification may be observed, and is relatively common around the finger joints.

Internal organ symptoms occur in the gastrointestinal tract, lungs, heart or kidneys. In the gastrointestinal tract, the esophagus is the most commonly affected site, accounting for more than 90% of cases. Peristalsis of the esophagus, dysphagia and reflux esophagitis commonly occur. Symptoms such as constipation, diarrhea and malabsorption may occur due to decreased small intestinal motility. The most important cause of death in patients with systemic sclerosis is pulmonary symptoms, which occur in approximately 70% of patients. Due to pulmonary fibrosis, patients complain of dyspnea and cough during exercise. Alveolitis frequently occurs, which continuously reduces pulmonary function. Cardiac symptoms include cardiac conduction disturbances, heart failure and pericarditis, and myocardial sclerosis may occur in 50 to 70% of patients. Renal symptoms occur in approximately 45% of patients with systemic sclerosis. They may manifest as sudden acute renal failure and hypertension, and slowly progressive uremia may occur.

Therefore, in the use of the present invention for preventing, ameliorating or treating skin fibrosis, skin fibrosis includes, without limitation, fibrosis of internal cavities of organs or glands such as blood vessels and veins, ducts of submandibular, gall bladder, thyroid follicles, sweat gland ducts, ovaries and kidneys; epithelial cells of the gums, tongue, palate, nose, larynx, esophagus, stomach, intestines, rectum, anus and vagina; fibrosis of any skin tissue and epithelial cells including the dermis, scar, skin and scalp.

In the use of the composition of the present invention for preventing, ameliorating or treating fibrosis, a fifth aspect relates to cardiac fibrosis, which is fibrosis occurring in the heart.

The term "cardiac fibrosis" in the present invention refers to a phenomenon in which the heart hardens due to excessive deposition of matrix proteins between cardiac cells, which mainly occurs in the hearts of myocardial infarction patients and is the main cause of decreased cardiac function, and may include, but is not limited to, any one or more selected from the group consisting of endocardial fibrosis, atrial fibrosis, heart failure, myocardial infarction and cardiac fibrosis caused by metabolic diseases.

Fibrosis is characterized by imbalanced accumulation of fibrous collagen following cardiomyocyte death, inflammation, increased workload, hypertrophy, and stimulation by multiple hormones, cytokines and growth factors.

Cardiac fibrosis may also refer to abnormal thickening of the heart valves due to the imbalanced proliferation of cardiac fibroblasts, but more commonly refers to the proliferation of fibroblasts in the heart muscle. Fibrocyte cells normally secrete collagen and provide structural support for the heart. When overly activated, this process causes thickening and fibrosis of the valves.

Since cardiac fibrosis is a major cause of heart failure and myocardial infarction, the term "cardiac fibrosis" may be interpreted to encompass heart failure and/or myocardial infarction caused by cardiac fibrosis.

In the use of the composition of the present invention for preventing, ameliorating or treating fibrosis, a sixth aspect relates to fatty fibrosis.

As described above, the present invention has confirmed that NOV-JD-1 exhibits anti-fibrotic activity by reducing the expression of various fibrosis marker genes or proteins in various tissue cells and animal models in which fibrosis is induced. Therefore, it can be expected that it may be effectively applied to the prevention, amelioration or treatment of fibrosis occurring in various tissues and/or organs in the body, including the liver, lungs, skin, kidney, heart, pancreas, nerves, muscles and joints.

In the composition for preventing, ameliorating or treating fibrosis of the present invention, the terms "prevention", "amelioration" and/or "treatment" mean any act that inhibits or delays the onset of a disease or condition, any act that improves or beneficially alters the state of a disease or condition, and any act that delays, stops or reverses the progression of a disease or condition.

As used herein, the term "pharmaceutically acceptable" means physiologically acceptable and does not typically cause an allergic reaction or similar reaction when administered to a human, and the salt is preferably an acid adduct formed by a pharmaceutically acceptable free acid.

The pharmaceutically acceptable salt may be an acid addition salt formed using an organic acid or an inorganic acid, and the organic acid includes, for example, formic acid, acetic acid, propionic acid, lactic acid, butyric acid, isobutyric acid, trifluoroacetic acid, malic acid, maleic acid, malonic acid, fumaric acid, succinic acid, succinic acid monoamide, glutamic acid, tartaric acid, oxalic acid, citric acid, glycolic acid, glucuronic acid, ascorbic acid, benzoic acid, phthalic acid, salicylic acid, anthranilic acid, dichloroacetic acid, aminooxy acetic acid, benzenesulfonic acid, p-toluenesulfonic acid or methanesulfonic acid. The inorganic acid includes, for example, hydrochloric acid, hydrobromic acid, sulfuric acid, phosphoric acid, nitric acid, carbonic acid or boric acid. The acid addition salt may preferably be in the form of a hydrochloride or an acetate salt, more preferably in the form of a hydrochloride salt.

In addition, additional possible salt forms include gabapentin salt, gabapentin salt, pregabalin salt, nicotinate salt, adipate salt, hemimalonate salt, cysteine salt, acetylcysteine salt, methionine salt, arginine salt, lysine salt, ornithine salt or aspartate salt, etc.

In addition, the pharmaceutical composition of the present invention may further include a pharmaceutically acceptable carrier. The pharmaceutically acceptable carrier may further include, for example, a carrier for oral administration or a carrier for parenteral administration. The carrier for oral administration may include lactose, starch, cellulose derivatives, magnesium stearate, stearic acid and the like. The carrier for parenteral administration may include water, suitable oils, saline, aqueous glucose and glycols and the like. In addition, the composition may further include a stabilizer and a preservative. Suitable stabilizers include antioxidants such as sodium bisulfite, sodium sulfite or ascorbic acid. Suitable preservatives include benzalkonium chloride, methyl- or propyl-paraben and chlorobutanol. Other pharmaceutically acceptable carriers may be referred to those described in the following literature (Remington's Pharmaceutical Sciences, 19th ed., Mack Publishing Company, Easton, PA, 1995).

The pharmaceutical composition of the present invention may be administered to mammals, including humans, by any method. For example, it may be administered orally or parenterally, and parenteral administration methods include, but are not limited to, intravenous, intramuscular, intraarterial, intramedullary, intrathecal, intracardiac, transdermal, subcutaneous, intraperitoneal, intranasal, enteral, topical, sublingual or rectal administration.

The pharmaceutical composition of the present invention may be formulated as a preparation for oral administration or parenteral administration according to the administration route as described above. When formulated, it may be prepared using one or more buffers (e.g., saline or PBS), carbohydrates (e.g., glucose, mannose, sucrose, or dextran, etc.), antioxidants, bacteriostats, chelating agents (e.g., EDTA or glutathione), fillers, bulking agents, binders, adjuvants (e.g., aluminum hydroxide), suspending agents, thickening agents, wetting agents, disintegrating agents or surfactants, diluents or excipients.

Solid preparations for oral administration include tablets, pills, powders, granules, liquids, gels, syrups, slurries, suspensions or capsules, and these solid preparations may be prepared by mixing the pharmaceutical composition of the present invention with at least one excipient, for example, starch (including corn starch, wheat starch, rice starch, potato starch, etc.), calcium carbonate, sucrose, lactose, dextrose, sorbitol, mannitol, xylitol, erythritol maltitol, cellulose, methyl cellulose, sodium carboxymethyl cellulose and hydroxypropyl methyl-cellulose or gelatin. For example, a tablet or a sugar-coated tablet may be obtained by mixing an active ingredient with a solid excipient, grinding the mixture, adding a suitable auxiliary agent, and then processing the same into a granule mixture.

In addition to simple excipients, lubricants such as magnesium stearate and talc are also used. Liquid preparations for oral administration include suspensions, solutions, emulsions or syrups, and may include various excipients such as wetting agents, sweeteners, flavoring agents or preservatives in addition to the commonly used simple diluents such as water or liquid paraffin.

In addition, cross-linked polyvinylpyrrolidone, agar, alginic acid or sodium alginate may be added as a disintegrating agent depending on the case, and anticoagulants, lubricants, wetting agents, fragrances, emulsifiers and preservatives may be additionally included.

When administered parenterally, the pharmaceutical composition of the present invention may be formulated in the form of injections, transdermal administration agents and nasal inhalers together with a suitable parenteral carrier according to methods known in the art. In the case of the injection, it must be sterilized and protected from contamination by microorganisms such as bacteria and fungi. Examples of suitable carriers for injection include, but are not limited to, solvents or dispersion media including water, ethanol, polyols (e.g., glycerol, propylene glycol, and liquid polyethylene glycol), mixtures thereof and/or vegetable oils. More preferably, suitable carriers include Hanks' solution, Ringer's solution, phosphate buffered saline (PBS) containing triethanolamine or isotonic solutions such as sterile water for injection, 10% ethanol, 40% propylene glycol and 5% dextrose. In order to protect the injection from microbial contamination, various antibacterial and antifungal agents such as paraben, chlorobutanol, phenol, sorbic acid, thimerosal and the like may be additionally included. In addition, the injection may in most cases additionally include an isotonic agent such as sugar or sodium chloride.

In the case of percutaneous administration, the forms include ointments, creams, lotions, gels, external solutions, pastes, liniments, aerosols, and the like. As used above, 'percutaneous administration' means topically administering a pharmaceutical composition to the skin such that an effective amount of active ingredients contained in the pharmaceutical composition are delivered into the skin.

For inhalation administration, the compounds used according to the present invention may conveniently be delivered in the form of an aerosol spray from a pressurized pack or nebulizer, using a suitable propellant, for example, dichlorofluoromethane, trichlorofluoromethane, dichlorotetrafluoroethane, carbon dioxide or other suitable gas. In the case of a pressurized aerosol, the dosage unit may be determined by providing a valve which delivers a metered amount. For example, gelatin capsules and cartridges for use in an inhaler or insufflator may be formulated by containing a powder mixture of the compound and a suitable powder base such as lactose or starch. Formulations for parenteral administration are described in the well-known prescription book of pharmaceutical chemistry (Remington's Pharmaceutical Science, 15th Edition, 1975. Mack Publishing Company, Easton, Pennsylvania 18042, Chapter 87: Blaug, Seymour).

The pharmaceutical composition of the present invention may provide a desirable effect of preventing, ameliorating or treating fibrosis when it includes an effective amount of a cyclo-hispro derivative. As used herein, the term "effective amount" refers to an amount that exhibits a greater response than a negative control, and preferably refers to an amount sufficient to prevent, ameliorate or treat fibrosis. The pharmaceutical composition of the present invention may include 0.01 to 99.9% of the cyclo-hispro derivative, and the remainder may be included of a pharmaceutically acceptable carrier. The effective amount of the cyclo-hispro derivative included in the pharmaceutical composition of the present invention will vary depending on the form in which the composition is commercialized

The total effective amount of the pharmaceutical composition of the present invention may be administered to a patient as a single dose, or may be administered by a fractionated treatment protocol in which multiple doses are administered for a long period of time. The pharmaceutical composition of the present invention may vary the content of the effective ingredient depending on the degree of the disease. For example, based on the cyclo-hispro derivative, it may be administered in one to several divided doses such that it is preferably administered in an amount of 0.001 to 100 mg, and more preferably 0.01 to 10 mg per kg of body weight per day. However, since the dosage of the cyclo-hispro derivative is determined by considering various factors such as the administration route and the number of treatments of the pharmaceutical composition as well as the patient's age, weight, health condition, sex, severity of the disease, diet and excretion rate, a person having ordinary knowledge in the art will be able to determine an appropriate effective dosage of the cyclo-hispro derivative according to a specific use for preventing, treating or ameliorating fibrosis. The pharmaceutical composition according to the present invention is not particularly limited in its formulation, administration route and administration method as long as it exhibits the effects of the present invention.

The pharmaceutical composition for preventing or treating fibrosis of the present invention may be used alone or in combination with methods using surgery, radiation therapy, hormone therapy, chemotherapy or biological response modifiers.

The pharmaceutical composition for preventing or treating fibrosis of the present invention may also be provided in the form of an external preparation including a cyclo-hispro derivative. In this respect, the composition of the present invention may be a quasi-drug composition for preventing or ameliorating fibrosis and a quasi-drug product including the composition.

The external preparation may be directly applied to the skin or oral cavity. When the pharmaceutical composition for preventing or treating fibrosis of the present invention is used as an external preparation, it may additionally contain adjuvants commonly used in the field of dermatology, such as fatty substances, organic solvents, solubilizers, thickening and gelling agents, softeners, antioxidants, suspending agents, stabilizers, foaming agents, fragrances, surfactants, water, ionic emulsifiers, nonionic emulsifiers, fillers, metal ion sequestering agents, chelating agents, preservatives, vitamins, blocking agents, humectants, essential oils, dyes, pigments, hydrophilic active agents, lipophilic active agents or lipid vesicles. In addition, the above components may be introduced in an amount commonly used in the field of dermatology.

When the composition of the present invention is provided as an external agent, it may be in the form of a liquid, ointment, patch, gel, cream or spray, but is not limited thereto. According to one embodiment of the present invention, the external medicine of the present invention may include oral care products including toothpaste, mouthwash, and mouth spray, ointments, masks, wet compresses, patches and transdermal absorbents.

When the composition of the present invention is used as a quasi-drug composition, the cyclo-hispro derivative may be added as is or may be used appropriately according to a conventional method together with other quasi-drug ingredients. The mixing amount of the effective ingredient may be appropriately determined depending on the purpose of use (prevention, health or therapeutic treatment).

The contents of the pharmaceutical composition and health functional food composition of the present invention may be applied to the pharmaceutical composition and the health functional food of the present invention.

In the present invention, the term "sitologically acceptable" means physiologically acceptable and does not typically cause an allergic reaction or similar reaction when ingested by humans, and the salt is preferably an acid addition salt formed by a sitologically acceptable free acid.

In the present invention, preferred examples of "sitologically acceptable salts" may include the types of "pharmaceutically acceptable salts" described above.

In the present invention, the term "health functional food" includes both the meanings of "functional food" and "health food".

In the present invention, the term "health functional food" includes both the meanings of "functional food" and "health food".

In the present invention, the term "functional food" is the same as food for special health use (FoSHU), and means a food with high medical and healthcare effects that is processed to efficiently exhibit a bioregulatory function in addition to providing nutrition.

In the present invention, the term "health food" means a food that has an active health maintenance or promotion effect compared to general food, and health supplement food means a food for the purpose of health supplementation. In some cases, the terms functional food, health food and health supplement food are used interchangeably. The food may be manufactured in various forms such as tablets, capsules, powders, granules, liquids and pills in order to obtain a useful effect in improving and recovering liver function.

As a specific example of such functional foods, processed foods may be manufactured by using the composition to transform and preserve the characteristics of agricultural, livestock or marine products while improving their storage properties.

The health functional food composition of the present invention may also be manufactured in the form of a nutritional supplement, a food additive, a feed and the like, and is intended for consumption by animals including humans or livestock.

The food composition of the above type may be manufactured in various forms according to conventional methods known in the art. General foods include, but are not limited to, beverages (including alcoholic beverages), fruits and processed foods thereof (e.g., canned fruits, bottled fruits, jams, marmalades, etc.), fish, meats and processed foods thereof (e.g., ham, sausages, corned beef, etc.), breads and noodles (e.g., udon, buckwheat noodles, ramen, spaghetti, macaroni, etc.), fruit juices, various drinks, cookies, taffy, dairy products (e.g., butter, cheese, etc.), edible plant fats, margarine, vegetable proteins, retort foods, frozen foods, various seasonings (e.g., soybean paste, soy sauce, sauces, etc.) and the like, which can be manufactured by adding a cyclo-hispro derivative thereto.

In addition, nutritional supplements may be manufactured by adding a cyclo-hispro derivative or a sitologically acceptable salt thereof to capsules, tablets, pills and the like, but are not limited thereto.

In addition, as a health functional food, it is not limited thereto, but for example, the cyclo-hispro derivative or sitologically acceptable salt thereof may be manufactured in the form of tea, juice and drink and may be consumed by liquefying, granulating, encapsulating, and powdering such that it can be consumed (health beverage). Additionally, in order to use the cyclo-hispro derivative or sitologically acceptable salt in the form of a food additive, it may be manufactured in the form of a powder or concentrate and used. In addition, it may be manufactured in the form of a composition by mixing the cyclo-hispro derivative with a known active ingredient known to be effective in preventing or ameliorating fibrosis.

When the food composition of the present invention is used as a health beverage composition, the health beverage composition may contain various flavoring agents or natural carbohydrates as additional ingredients, like a typical beverage. The above-mentioned natural carbohydrates may be monosaccharides such as glucose and fructose; disaccharides such as maltose and sucrose; polysaccharides such as dextrin and cyclodextrin; and sugar alcohols such as xylitol, sorbitol and erythritol. The sweetener may be a natural sweetener such as thaumatin and stevia extract; a synthetic sweetener such as saccharin and aspartame and the like. The proportion of the natural carbohydrate is generally about 0.01 to 0.04 g, and preferably about 0.02 to 0.03 g, per 100 mL of the composition of the present invention.

The cyclo-hispro derivative or sitologically acceptable salt of the present invention may be contained as an effective ingredient of a food composition for preventing or ameliorating fibrosis, and the amount thereof is an amount effective to obtain the above-mentioned preventive or improving effect, for example, preferably 0.01 to 100 wt% based on the total weight of the entire composition, but is not particularly limited thereto. The food composition of the present invention may be prepared by mixing the cyclo-hispro derivative or sitologically acceptable salt thereof with another active ingredient known to be effective in preventing or ameliorating fibrosis.

In addition to the above, the health functional food of the present invention may contain various nutrients, vitamins, electrolytes, flavoring agents, coloring agents, pectic acid, salts of pectic acid, alginic acid, salts of alginic acid, organic acids, protective colloid thickeners, pH regulators, stabilizers, preservatives, glycerin, alcohols or carbonating agents. In addition, the health functional food of the present invention may contain fruit pulp for the production of natural fruit juice, fruit juice drinks or vegetable drinks. These ingredients may be used independently or in mixtures. The ratio of these additives is not particularly important, but is generally selected in the range of 0.01 to 0.1 parts by weight per 100 parts by weight of the composition of the present invention.

The present invention also relates to a method for preventing, ameliorating or treating fibrosis, including administering an effective amount of a cyclo-hispro derivative or a pharmaceutically acceptable salt thereof to a subject in need thereof.

In the method of the present invention, the term "subject" includes, but is not limited to, any animal (e.g., human, horse, pig, rabbit, dog, sheep, goat, non-human primate, cow, cat, guinea pig, or rodent). This term does not indicate a particular age or sex. Therefore, it is intended to include female/female or male/male, adult/adult and neonatal subjects, as well as fetuses. A patient refers to a subject suffering from a disease or disorder. The term patient includes human and veterinary subjects.

In the method of the present invention, the description of the composition including the effect of the cyclo-hispro derivative, its administration route, number of administrations, dosage and the like, is the same as described above, and therefore, the description thereof is omitted.

The present invention also provides the use of a cyclo-hispro derivative or a pharmaceutically acceptable salt thereof in the manufacture of a medicament or health functional food for preventing, ameliorating or treating fibrosis.

### MODES OF THE INVENTION

Hereinafter, the present invention will be described in more detail through examples. However, the present invention may be modified in various ways and have various forms, and the specific examples and descriptions described below are only intended to help understand the present invention, and are not intended to limit the present invention to specific disclosed forms. It should be understood that the scope of the present invention includes all modifications, equivalents or substitutes included in the idea and technical scope of the present invention.

### [Example 1]

### Synthesis of (3S,8aS)-3-((1H-imidazol-4-yl-2-d)methyl)hexahydropyrrolo[1,2-a]pyrazine-1,4-dione

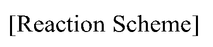

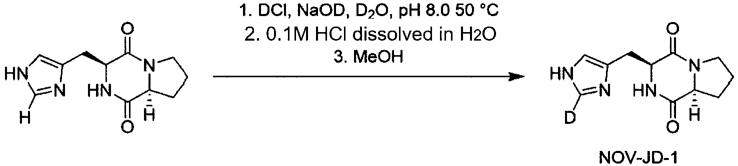

(3S,8aS)-3-((1H-imidazol-4-yl)methyl)hexahydropyrrolo[1,2-a]pyrazine-1,4-dione (Bachem) 3 g was dissolved in 10 mL of a solution adjusted to pH 8.0 in D₂O using a 20 wt% solution of deuterium chloride dissolved in D₂O and 40 wt% of sodium deuteroxide dissolved in D₂O, and the mixture was stirred at 80°C for 2 days. The solution was freeze-dried for 24 hours. Afterwards, the above procedure was repeated three times. The solid obtained through three repetitions was dissolved in 10 mL of 0.1 M HCl and stirred for 24 hours. After the above solution was lyophilized for 24 hours, it was dissolved in MeOH, filtered under reduced pressure, concentrated under reduced pressure, and dried under vacuum to obtain (3S,8aS)-3-((1H-imidazol-4-yl-2-d)methyl)hexahydropyrrolo[1,2-a]pyrazine-1,4-dione (NOV-JD-1) (yield 95%).

1H NMR (400 MHz, Methanol-d4) δ 7.07 - 6.83 (m, 1H), 4.15 - 4.07 (m, 1H), 3.55 (dd, J = 12.4, 8.1 Hz, 1H), 3.51 - 3.35 (m, 1H), 3.35 - 3.21 (m, 2H), 3.21 - 3.11 (m, 1H), 3.09 (dt, J = 7.2, 3.6 Hz, 1H), 3.01 (ddd, J = 14.5, 5.0, 1.9 Hz, 1H), 2.28 - 2.11 (m, 1H), 2.01 - 1.89 (m, 1H), 1.89 - 1.71 (m, 2H).

### [Example 2]

### Confirmation of anti-fibrotic effect of CHP derivative in liver cells

### 2-1. Cell culture

LX2 cells (Sigma-Aldrich SCC064), which are a human liver cell line, were cultured in DMEM medium (Hyclone) containing 10% FBS (Hyclone) and 1% penicillin/streptomycin antibiotics (Thermo) at 37°C in an incubator with 5% CO₂.

AML12 cells (ATCC CRL-2254), which are a mouse liver cell line, were cultured in DMEM/F12 medium (Thermo) containing 10% FBS, 1% antibiotics, and ITS (Insulin/Transferrin/Selenium; Thermo) and dexamethasone (Sigma) at 37°C in an incubator with 5% CO₂.

### 2-2. Induction of cell fibrosis and sample processing

LX2 cells were seeded in 6-well plates at a concentration of 2 × 10⁵ cells/well. After 24 hours, the cell medium was removed, and the negative control group was treated with serum-free medium, and the fibrosis-inducing groups were treated with NOV-JD-1 synthesized in Example 1 at concentrations of 0, 1, 5 and 25 µM using serum-free medium including TGF-β (R&D systems) at a concentration of 2 ng/mL, respectively. After 24 hours, the cells were washed once with cold PBS and recovered using a scraper.

AML12 cells were seeded in 6-well plates at a concentration of 2 × 10⁵ cells/well. After 24 hours, the cell medium was removed, and the negative control group was treated with serum-free medium, and the fibrosis-inducing groups were treated with NOV-JD-1 synthesized in Example 1 at concentrations of 0 and 10 µM using serum-free medium containing TGF-β at a concentration of 2 ng/mL, respectively. After 16 hours, the cells were washed once with cold PBS and recovered using a scraper.

### 2-3. Analysis of expression of intracellular fibrosis proteins

100 uL of RIPA lysis buffer (Thermo) containing protease and phosphatase inhibitors was added to each recovered cell, and the cells were lysed by pipetting. After incubating on ice for 10 minutes, the cells were centrifuged at 15,000 rpm for 10 minutes at 4°C. The supernatants were collected, and the protein concentration was measured by the BCA quantitation method using a BCA protein quantitation kit (Intron), and the same amount of samples was separated for protein using a Bolt^{TM} protein gel electrophoresis system (Thermo), and transferred to a nitrocellulose membrane. The membrane was blocked with 5% skim milk for 1 hour at room temperature and then reacted with primary antibodies, fibronectin antibody (Abcam), Gapdh antibody (Cell signaling) and vinculin antibody (Cell signaling) at 4°C overnight. After washing three times for 10 minutes each with TBST, it was reacted with secondary antibodies for 1 hour at room temperature. After washing three times for 10 minutes each with TBST, the expression level was measured by reacting with ECL. The density of the bands that appeared was quantified using the ImageJ program, and the fibronectin band density value was corrected by dividing it by the band density value of the loading control group.

As a result of the experiment, as shown in FIG. 1, the expression of fibronectin, which is a fibrosis protein increased by TGF-β in LX2 cells, was confirmed to be decreased in a concentration-dependent manner by 21.6, 20.1 and 37.3% in the groups treated with 1, 5 and 25 µM of NOV-JD-1, respectively, showing a concentration-dependent decrease. In addition, as shown in FIG. 2, the expression of fibronectin, which is a fibrosis protein increased by TGF-β in AML12 cells, was confirmed to be decreased by 48.2% in the group treated with 10 µM of NOV-JD-1.

The above results confirmed that NOV-JD-1 could ameliorate fibrosis in LX2 cells and AML12 cells, which are liver cell lines. Furthermore, since NOV-JD-1 showed an effect of ameliorating fibrosis in liver cell lines, it was expected that it could also ameliorate fibrosis of liver tissue and metabolic abnormality-associated steatohepatitis (MASH).

### [Example 3]

### Comparison of anti-fibrotic effects of CHP and CHP derivative in liver cells

In the same manner as in Example 2, the anti-fibrotic effects of CHP and NOV-JD-1 synthesized in Example 1 were compared in LX2 cells and AML12 cells induced with fibrosis.

As a result, as shown in FIG. 3, the expression of fibronectin, which is a fibrosis protein increased by TGF-β in LX2 cells, was confirmed to be reduced by 18.9 and 69.8% by CHP and 25 µM NOV-JD-1 treatment, respectively. The reduction by NOV-JD-1 showed a significant reduction of 62.8%, which is greater than that of CHP. Through this, it was confirmed that NOV-JD-1 can ameliorate fibrosis in LX2 cells, which are a liver cell line, and the effect thereof is superior to that of CHP. In addition, as shown in FIG. 4, the expression of fibronectin, which is a fibrosis protein increased by TGF-β in AML12 cells, was confirmed to be reduced by 23.9 and 70.6% by CHP and 10 µM NOV-JD-1 treatment, respectively. The reduction by NOV-JD-1 showed a significant reduction of 61.3%, which is greater than that of CHP. Through this, the inventors of the present invention confirmed that NOV-JD-1 can ameliorate fibrosis in AML12 cells, which are a liver cell line, and the effect thereof is superior to CHP.

Based on the above results, it was expected that NOV-JD-1 would be able to more effectively ameliorate fibrosis and metabolic abnormality-associated steatohepatitis (MASH) in liver tissue, as it showed an anti-fibrosis effect in liver cell lines and showed a significantly better effect than CHP.

### [Example 4]

### Confirmation of effects of CHP derivative in liver fibrosis/MASH animal model

### 4-1. Establishment of animal model for liver fibrosis/MASH induction

Nine-week-old male C57BL6/J mice (Daehan Biolink) were housed in an environment of 23 ± 3°C with free access to food (Purina) and water. After acclimating to the breeding environment for 1 week and measuring their body weight, the mice were randomly distributed to have uniform average body weights and divided into three groups as shown in Table 2. To induce liver fibrosis/MASH, 0.2 mL/kg (CCl4:olive oil = 1:19) of carbon tetrachloride (CCl₄) per mouse was intraperitoneally injected into the disease-induced and experimental groups on days 0, 2, 5, 7, 9, 12, 14, 16 and 19. Olive oil was injected in the same manner into the normal control group. NOV-JD-1 was orally administered daily at a concentration of 35 mg/kg. The normal control group and disease-induced group were orally administered the same amount of purified water (DW). Mice were euthanized 12 hour after the last CCl₄ injection, and blood and liver tissue were collected.

**[Table 1]**

| Group | Normal control group | Disease-induced group | Experimental group |
|---|---|---|---|
| CCl₄ | Olive oil | CCl₄ 0.2 mL/kg | |
| Administration | DW | DW | Nov-JD-1 35 mg/kg |
| | Oral administration | | |
| Number of mice | 4 | 8 | 8 |

### 4-2. Liver function test

For the liver function test of mice, blood was diluted 1:19 using the dilution buffer of the ALT assay kit (K-753) from Biovision and measured according to the kit protocol. As a result, as shown in FIG. 5, it was confirmed that the ALT activity increased by CCl₄ was significantly reduced by 13.6% by NOV-JD-1 administration.

### 4-3. Evaluation of gene expression in liver tissue

Liver tissues were extracted with NucleoZOL according to the manufacturer's (MACHEREY-NAGEL) total RNA isolation protocol, and 1 µg of RNA was subjected to reverse transcription polymerase chain reaction (RT-PCR) using ReverTra Ace qPCR RT Master Mix (Toyobo) to synthesize cDNA. The synthesized cDNA was analyzed by real-time PCR using primer sets of inflammatory cytokines *IL-1β and IL-6* genes and fibrotic genes *Collagen I, Collagen III and α-SMA* and SYBR Green Realtime PCR Master mix (Toyobo). The sequence information of the primers for real-time PCR is as shown in Table 2. The primers in Table 2 were synthesized by request to Bioneer. Each gene expression value was corrected by dividing it by the expression value of *gapdh*, which is a housekeeping gene.

**[Table 2]**

| Gene | Forward direction (5'-3') | SEQ ID NO: | Reverse direction (5'-3') | SEQ ID NO: |
|---|---|---|---|---|
| IL-1β | AAGGGCTGCTTCCAAACCTT TGAC | 1 | ATACTGCCTGCCTGAAG CTCTTGT | 2 |
| IL-6 | TTGCCTTCTTGGGACTGATG | 3 | TCCTCTGTGAAGTCTCC TCTC | 4 |
| Fibronectin | TTGGAGAGGAGTGGGAGC | 5 | GAAATGACCACTGCCA AAGC | 6 |
| Collagen I | GCCTCAGAAGAACTGGTAC AT | 7 | ATCCATCGGTCATGCTC TCT | 8 |
| Collagen III | AGTCAAGGAGAAAGTGGTC G | 9 | CCAGGGAAACCCATGA CAC | 10 |
| Collagen IV | CGGTACACAGTCAGACCATT | 11 | CATCACGAAGGAATAG CCGA | 12 |
| αSMA | GCATCCACGAAACCACCT | 13 | CAGGACGTTGTTAGCAT AGAGAT | 14 |
| Vimentin | GGATCAGCTCACCAACGAC A | 15 | ATTTCTCTCGCAGCCGC AT | 16 |
| β-actin | GGGAAGGTGACAGCATTG | 17 | ATGAAGTATTAAGGCG GAAGATT | 18 |
| Gapdh | CAGTATGACTCCACCCACGG | 19 | ATGGGCTTCCCGTTGAT GAC | 20 |

As a result of the experiment, as shown in FIG. 6, it was confirmed that the inflammatory cytokines *IL-1β and IL-6* increased by CCl₄ were significantly reduced by 46.3 and 45.0%, respectively, by NOV-JD-1 administration. In addition, as shown in FIG. 7, it was confirmed that the fibrosis genes *Collagen I, Collagen III* and *α-SMA* increased by CCl₄ were significantly reduced by 26.1, 23.6 and 40.7%, respectively, by NOV-JD-1 administration. Through the above results, it was confirmed that NOV-JD-1 can significantly reduce the expression of inflammation and fibrosis genes in liver tissue and significantly restore ALT liver function in liver fibrosis/MASH-induced mice, thereby improving MASH.

### [Example 5]

### Comparison of anti-fibrotic effects of CHP and CHP derivative in liver fibrosis/MASH animal model

In the same manner as in Example 4, the anti-fibrotic effects of CHP and NOV-JD-1 synthesized in Example 1 were compared in an animal model in which fibrosis was induced. Each group was divided into four groups as shown in Table 3.

**[Table 3]**

| Group | Normal control group | Disease-induced group | CHP group | NOV-JD-1 group |
|---|---|---|---|---|
| CCl₄ | Olive oil | CCl₄ 0. 2mL/kg | | |
| Administra | DW | DW | CHP | Nov-JD-1 |
| tion | | | 35 mg/kg | 35 mg/kg |
| | Oral administration | | | |
| Number of mice | 4 | 8 | 8 | 8 |

### 5-1. Measurement of hydroxyproline levels in liver tissue

The level of hydroxyproline, which is used as an indicator of collagen accumulation due to fibrosis, was measured using a hydroxyproline assay kit (MAK008, Sigma). As a result of measuring in the lung tissue of each group, as shown in FIG. 8, the level of hydroxyproline, which was rapidly increased by CCl₄, was significantly reduced by 10.1% by CHP administration and 26.2% by NOV-JD-1 administration. The reduction by NOV-JD-1 was confirmed to be 17.9% more than that by CHP, confirming that NOV-JD-1 ameliorates liver fibrosis in an animal model and that its effect is more effective than that of CHP.

### 5-2. Evaluation of gene expressions in liver tissue

The expression levels of the fibrosis gene α-SMA and the inflammatory cytokines *IL-1β* and *IL-6* genes were evaluated using the same method as in Example 4-3.

As a result of the experiment, as shown in FIG. 9, the expression of the fibrosis gene *α-SMA*, which was increased by CCl₄, was significantly reduced by 10.7% by CHP administration and by 36.9% by NOV-JD-1 administration. The reduction by NOV-JD-1 was also confirmed to be significantly reduced by 29.4% compared to CHP.

In addition, as shown in FIG. 10, the gene expression of inflammatory cytokines *IL-1β* (A) and *IL-6* (B), which were increased by CCl₄, was decreased by 13.4 and 8.6%, respectively, by CHP administration, and was further significantly decreased by 46.3 and 45.0%, respectively, by NOV-JD-1 administration. The decrease in the NOV-JD-1 administration group compared to the CHP administration group was also statistically significant.

### 5-3. Evaluation of expression of fibrotic proteins in liver tissue

Liver tissue was homogenized with 500 µL of RIPA buffer to which protease and phosphatase inhibitors were added, kept on ice for 15 minutes, and centrifuged at 15,000 rpm at 4°C for 10 minutes. The supernatant was collected to recover the lysate, and the subsequent Western blotting process was performed as in Example 2-3 using fibronectin and Gapdh primary antibodies.

As a result of the experiment, as shown in FIG. 10, the expression of fibronectin, which is a fibrous protein increased by CCl₄, was significantly reduced by 21.4% by CHP and by 49.6% by NOV-JD-1. Here again, the reduction by NOV-JD-1 was shown to be a significant decrease of 35.9% compared to CHP.

Through this, the inventors of the present invention confirmed that NOV-JD-1 could ameliorate liver fibrosis and MASH by significantly reducing the expression of fibrotic genes and inflammatory cytokine genes in liver tissue in liver fibrosis/MASH-induced mice and also significantly reducing the amounts of fibronectin and hydroxyproline, which are major fibrosis protein markers. In addition, the inventors of the present invention confirmed that the anti-fibrotic effect of NOV-JD-1 was more excellent than that of the same amount of CHP.

Statistical significance was analyzed using the t-test statistical method between groups. *p<0.05, **p<0.01, ***p<0.001, ****p<0.0001.

### [Example 6]

### Confirmation of anti-fibrotic effect of CHP derivative in lung cells

### 6-1. Cell culture

MRC5 cells (ATCC CCL-171), which are a human lung fibroblast cell line, were cultured in MEM medium containing 10% FBS and 1% antibiotics at 37°C in an incubator with 5% CO₂.

Additionally, to isolate primary lung fibroblasts from mice (Daehan Biolink Co.), the mice were anesthetized with 3% isoflurane, then, laparotomy was performed, the aorta was cut, and 10 mL of PBS was injected into the right ventricle to perfuse and wash the lungs. The lungs were then taken out, washed with cold PBS, and finely ground with a scalpel. The finely ground tissues were dispersed in DMEM medium containing 10% FBS and 1% antibiotics and evenly spread in a 150 mm culture dish. The medium was changed once every two days while culturing in an incubator at 37°C and 5% CO₂. On the 6th to 7th day, only the attached cells were isolated and cultured on a new culture dish.

### 6-2. Induction of cell fibrosis and sample processing

MRC5 cells were seeded in 6-well plates at a concentration of 2 × 10⁵ cells/well. After 24 hours, the medium was removed, and the negative control group was treated with serum-free medium, and the fibrosis-induced groups were treated with serum-free medium including TGF-β at a concentration of 2 ng/mL, and NOV-JD-1 was treated at concentrations of 0 and 10 µM, respectively. After 16 hours, the cells were washed once with cold PBS and recovered using a scraper.

Primary lung fibroblasts isolated from mice were seeded in 6-well plates at a density of 2 × 10⁵ cells/well. After 24 hours, the medium was removed, and the negative control group was treated with serum-free medium, and the fibrosis-induced groups were treated with NOV-JD-1 at concentrations of 0 and 25 µM using serum-free medium including TGF-β at a concentration of 5 ng/mL, respectively. After 48 hours, the cells were washed once with cold PBS and recovered using a scraper.

### 6-3. Analysis of expression of intracellular fibrosis proteins

The expression of fibrosis proteins was analyzed in the same manner as in Example 2-3. As a result of the experiment, as shown in FIG. 11, it was confirmed that the expression of fibrosis protein fibronectin, which increases by TGF-β in MRC5 cells, was significantly reduced by 22% in the group treated with 10 µM NOV-JD-1. In addition, as shown in FIG. 12, it was confirmed that the expression of fibrosis protein Collagen I, which increases by TGF-β in mouse primary lung fibroblasts, was significantly reduced by 32% in the group treated with 25 µM NOV-JD-1.

The above results confirmed that NOV-JD-1 could ameliorate fibrosis in MRC5 cells, which are a lung cell line, and mouse primary lung fibroblasts. As such, since NOV-JD-1 showed an anti-fibrosis effect in lung cells, it was expected that it could also ameliorate fibrosis in lung tissue.

### [Example 7]

### Confirmation of anti-fibrotic effects of CHP and CHP derivative in lung cells

The anti-fibrotic effects of CHP and NOV-JD-1 synthesized in Example 1 were compared in mouse primary lung fibroblasts induced to undergo fibrosis in the same manner as in Example 6.

As a result of analyzing the expression of intracellular fibrosis proteins, as shown in FIG. 13, the expression of fibrosis protein fibronectin, which is increased by TGF-β in mouse primary lung fibroblasts, was significantly reduced by 27% by CHP and by 53.8% by NOV-JD-1. The reduction by NOV-JD-1 was confirmed to be 36.7% greater than that by CHP. Through this, it was expected that NOV-JD-1 could ameliorate fibrosis with a better effect than CHP in mouse primary lung fibroblasts and could also ameliorate fibrosis of animal lung tissue.

### [Example 8]

### Confirmation of effects of CHP derivative in animal model of idiopathic pulmonary fibrosis

### 8-1. Establishment of animal model of idiopathic pulmonary fibrosis

C57BL6/J mice were housed in an environment of 23 ± 3°C after introduction, with free access to food and water. After acclimating to the housekeeping environment for 1 week, 0.03 IU/50µL of bleomycin per subject was injected intratracheally using a catheter to induce IPF. The same amount of saline was injected intratracheally to the normal control group. After 1 week, the body weights were measured, and the mice were randomly distributed to have equal average body weights and divided into three groups as shown in Table 4. The normal control group and the disease-induced group were orally administered DW daily for 2 weeks starting from 1 week after bleomycin injection, and the experimental group was orally administered NOV-JD-1 at a concentration of 35 mg/kg for the same period.

**[Table 4]**

| Group | Normal control group | Disease-induced group | Experimental group |
|---|---|---|---|
| Bleomycin (BLM) | Saline solution | BLM 0.03 IU/head | |
| Administration | DW | DW | Nov-JD-1 25mg/kg |
| | Oral administration | | |
| Number of mice | 4 | 7 | 8 |

### 8-2. Measurement of hydroxyproline levels in lung tissue

The level of hydroxyproline, which is used as an indicator of collagen accumulation due to fibrosis, was measured in the lung tissue of each group. As shown in FIG. 14, it was confirmed that the level of hydroxyproline, which was rapidly increased by BLM, was significantly reduced by 32.4% by NOV-JD-1 administration.

### 8-3. Evaluation of gene expression in lung tissue

The expression levels of the fibrosis genes *Fibronectin, Collagen I, Collagen III,* and *Collagen IV* in lung tissue were evaluated using the primers in Table 2 in the same manner as in Example 4-3. As a result of the experiment, as shown in FIG. 15, it was confirmed that the fibrosis genes *Fibronectin, Collagen I, Collagen III,* and *Collagen IV,* which were increased by BLM, were significantly reduced by 35.2, 35.5, 32.5 and 48.1%, respectively, by NOV-JD-1 administration.

### 8-4. Evaluation of fibrotic protein expression in lung tissue

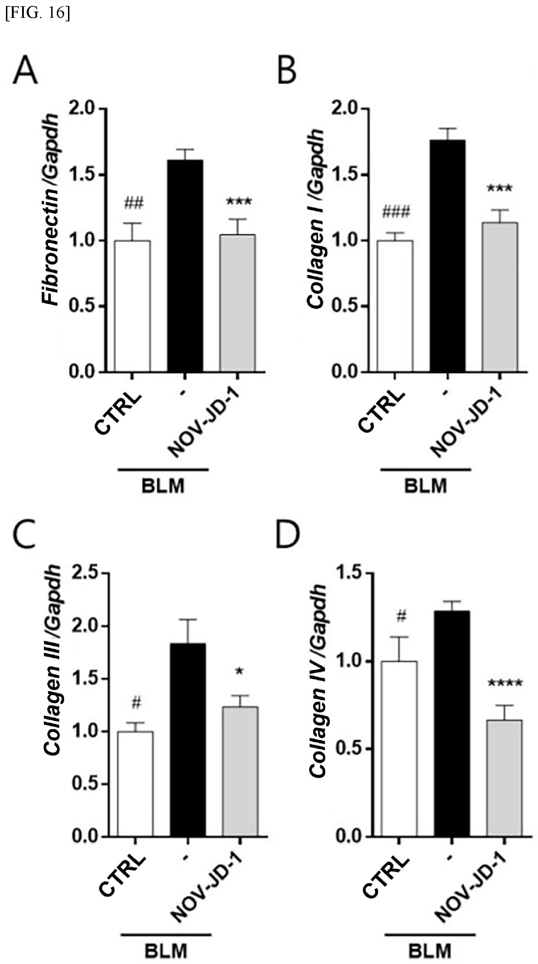

The expression of fibronectin, which is a fibrotic protein, in lung tissue was analyzed using the same method as in Example 2-3. As a result of the experiment, as shown in FIG. 16, it was confirmed that the expression level of fibronectin protein increased by BLM was significantly reduced by 52.8% by NOV-JD-1 administration.

Through the above results, the inventors of the present invention confirmed that NOV-JD-1 can ameliorate IPF as several fibrosis markers in lung tissue were significantly reduced by administration of NOV-JD-1 in a BLM-induced IPF mouse model.

### [Example 9]

### Comparison of anti-fibrotic effects of CHP and CHP derivative in animal model of idiopathic pulmonary fibrosis

In an animal model of idiopathic pulmonary fibrosis, the same method as in Example 8 was used to compare the anti-fibrosis effects of CHP and NOV-JD-1 synthesized in Example 1. Each group was divided into four groups as shown in Table 5.

**[Table 5]**

| Group | Normal control group | Disease-induced group | CHP group | Experimental group |
|---|---|---|---|---|
| Bleomycin (BLM) | Saline solution | BLM 0.03 IU/head | | |
| Administration | DW | DW | CHP 25mg/kg | Nov-JD-1 25mg/kg |
| | Oral administration | | | |
| Number of mice | 4 | 7 | 8 | 8 |

### 9-1. Evaluation of gene expression in lung tissue

The expression levels of α-SMA and *Vimentin* genes, which are fibrosis genes, in lung tissue were evaluated using the primers in Table 2 in the same manner as in Example 4-3. Each gene expression value was corrected by dividing it by the expression values of *β-actin* and *gapdh*, which are housekeeping genes.

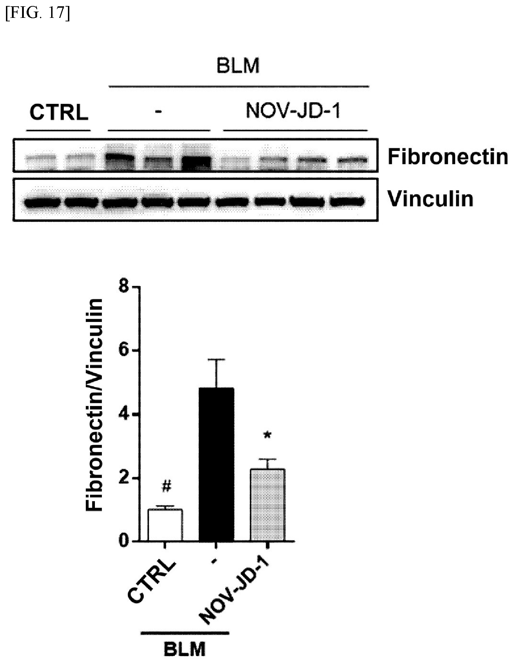

As shown in FIG. 17, the expression of fibrosis genes *αSMA* (A) and *Vimentin* (B) increased by BLM was decreased by 33% and 25.4%, respectively, by CHP administration, and was further significantly decreased by 52.5% and 50.8%, respectively, by NOV-JD-1 administration. This confirmed that NOV-JD-1 can ameliorate IPF much more effectively than CHP in the BLM-induced IPF mouse model.

Statistical significance was analyzed using the t-test statistical method between groups. *p<0.05, **p<0.01.

## Claims

1. A compound represented by Chemical Formula 1 below, a stereoisomer thereof or a pharmaceutically acceptable salt thereof:

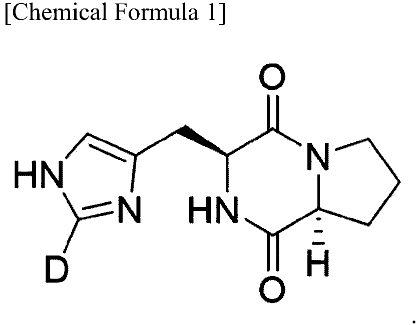

2. A pharmaceutical composition for preventing or treating fibrosis, comprising a compound represented by Chemical Formula 1 below, a stereoisomer thereof or a pharmaceutically acceptable salt thereof:

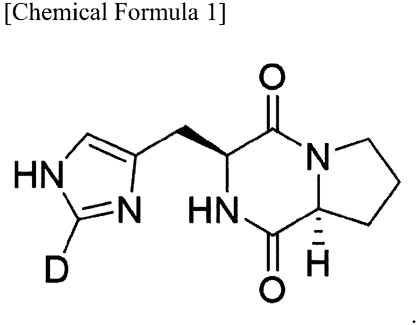

3. The pharmaceutical composition of claim 2, wherein the fibrosis is any one or more selected from the group consisting of hepatic fibrosis, pulmonary fibrosis, renal fibrosis, dermal fibrosis, cardiac fibrosis, pancreatic fibrosis, neural fibrosis, muscular fibrosis and articular fibrosis.

4. The pharmaceutical composition of claim 3, wherein the hepatic fibrosis is caused by any one or more selected from the group consisting of chronic liver disease, hepatitis B virus infection, hepatitis C virus infection, hepatitis D virus infection, schistosomiasis, alcoholic liver disease, metabolic dysfunction-associated steatohepatitis, metabolic disease, protein deficiency, coronary artery disease, autoimmune hepatitis, cystic fibrosis, alpha-1 antitrypsin deficiency, primary biliary cirrhosis, drug reaction and toxins.

5. The pharmaceutical composition of claim 3, wherein the pulmonary fibrosis is caused by any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis, diffuse pulmonary fibrosis, pulmonary edema, cystic fibrosis and metabolic disease.

6. A health functional food composition for preventing or ameliorating fibrosis, comprising a compound represented by Chemical Formula 1 or a sitologically acceptable salt thereof:

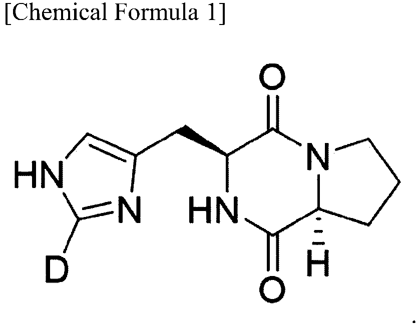

7. The health functional food composition of claim 6, wherein the fibrosis is any one or more selected from the group consisting of hepatic fibrosis, pulmonary fibrosis, renal fibrosis, dermal fibrosis, cardiac fibrosis, pancreatic fibrosis, neural fibrosis, muscular fibrosis and articular fibrosis.

8. The health functional food of claim 7, wherein the hepatic fibrosis is caused by any one or more selected from the group consisting of chronic liver disease, hepatitis B virus infection, hepatitis C virus infection, hepatitis D virus infection, schistosomiasis, alcoholic liver disease, metabolic dysfunction-associated steatohepatitis, metabolic disease, protein deficiency, coronary artery disease, autoimmune hepatitis, cystic fibrosis, alpha-1 antitrypsin deficiency, primary biliary cirrhosis, drug reaction and toxins.

9. The health functional food composition of claim 7, wherein the pulmonary fibrosis is caused by any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis, diffuse pulmonary fibrosis, pulmonary edema, cystic fibrosis and metabolic disease.

10. An anti-fibrotic composition, comprising a compound represented by Chemical Formula 1 below or a pharmaceutically acceptable salt thereof:

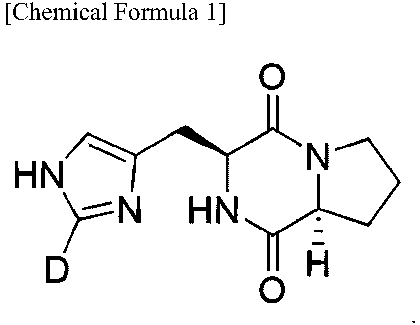

11. A method for preventing, ameliorating or treating fibrosis, comprising administering an effective amount of a compound represented by Chemical Formula 1 below or a pharmaceutically acceptable salt thereof to a subject in need thereof:

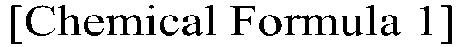

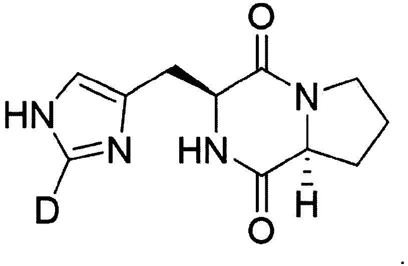

12. The method of claim 11, wherein the fibrosis is any one or more selected from the group consisting of hepatic fibrosis, pulmonary fibrosis, renal fibrosis, dermal fibrosis, cardiac fibrosis, pancreatic fibrosis, neural fibrosis, muscular fibrosis and articular fibrosis.

13. The method of claim 12, wherein the hepatic fibrosis is caused by any one or more selected from the group consisting of chronic liver disease, hepatitis B virus infection, hepatitis C virus infection, hepatitis D virus infection, schistosomiasis, alcoholic liver disease, metabolic dysfunction-associated steatohepatitis, metabolic disease, protein deficiency, coronary artery disease, autoimmune hepatitis, cystic fibrosis, alpha-1 antitrypsin deficiency, primary biliary cirrhosis, drug reaction and toxins.

14. The method of claim 12, wherein the pulmonary fibrosis is caused by any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis, diffuse pulmonary fibrosis, pulmonary edema, cystic fibrosis and metabolic disease.

15. Use of a compound represented by Chemical Formula 1 below or a pharmaceutically or sitologically acceptable salt thereof in the manufacture of a medicament or health functional food for preventing, ameliorating or treating fibrosis:

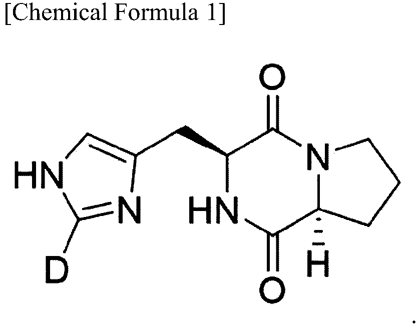

16. The use of claim 15, wherein the fibrosis is any one or more selected from the group consisting of hepatic fibrosis, pulmonary fibrosis, renal fibrosis, dermal fibrosis, cardiac fibrosis, pancreatic fibrosis, neural fibrosis, muscular fibrosis and articular fibrosis.

17. The use of claim 16, wherein the hepatic fibrosis is caused by any one or more selected from the group consisting of chronic liver disease, hepatitis B virus infection, hepatitis C virus infection, hepatitis D virus infection, schistosomiasis, alcoholic liver disease, metabolic dysfunction-associated steatohepatitis, metabolic disease, protein deficiency, coronary artery disease, autoimmune hepatitis, cystic fibrosis, alpha-1 antitrypsin deficiency, primary biliary cirrhosis, drug reaction and toxins.

18. The use of claim 16, wherein the pulmonary fibrosis is caused by any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis, diffuse pulmonary fibrosis, pulmonary edema, cystic fibrosis and metabolic disease.
